# EUROPÄISCHE PATENTSCHRIFT

(11) **EP 2 166 968 B1**
(45) Veröffentlichungstag und Bekanntmachung des Hinweises auf die Patenterteilung: **04.05.2011**
(21) Anmeldenummer: 08758617.8
(22) Anmeldetag: 19.05.2008
(51) Int. Cl.: A61B 17/32, A61B 10/02, A61B 17/34, F16H 3/14

(54) **VORRICHTUNG ZUM AUSSCHNEIDEN UND ENTFERNEN VON GEWEBEZYLINDERN AUS EINEM GEWEBE UND DEREN VERWENDUNG**
APPARATUS FOR CUTTING OUT AND REMOVING TISSUE CYLINDERS FROM A TISSUE, AND USE THEREOF
DISPOSITIF DE DÉCOUPE ET DE RETRAIT DE CYLINDRES À TISSUS, À PARTIR D'UN TISSU ET SON UTILISATION

(30) Priorität: 18.05.2007 DE 102007023207; 17.10.2007 DE 102007049796
(43) Veröffentlichungstag der Anmeldung: 31.03.2010
(73) Patentinhaber: WISAP Gesellschaft für wissenschaftlichen Apparatebau mbH, 82054 Sauerlach (DE)
(72) Erfinder: REPPENTHIEN, Joachim, 82054 Sauerlach (DE)
(74) Vertreter: Grape, Knut
(86) Internationale Anmeldenummer: PCT/EP2008/004001
(87) Internationale Veröffentlichungsnummer: WO 2008/141791

(56) Entgegenhaltungen:
- WO-A-99/07295
- US-A- 3 778 179
- US-A- 4 021 126
- US-A- 4 306 570
- US-A- 5 324 300
- US-A- 5 947 852

## Beschreibung

Die Erfindung betrifft eine Vorrichtung zum Ausschneiden und Entfernen von Gewebezylindern aus einem Gewebe, das sich innerhalb einer Körper- oder Gelenkhöhle und/oder in bzw. an einem Wandbereich davon befindet.

Derartige Vorrichtungen sind allgemein bekannt. Demnach dienen solche Vorrichtungen unter gleichzeitiger Verwendung eines Trokars und einer Trokarhülse beispielsweise auf dem medizinischen Gebiet der Gynäkologie einem Entfernen von Gewebe aus einer Gebärmutter, einer Gebärmutter insgesamt oder zur Behandlung von Myomen, etc. Aus der EP 0 522 125 B1 ist zum Beispiel eine derartige Vorrichtung bekannt. Derartige Vorrichtungen finden darüber hinaus in der Medizin auf zahlreichen und zum Teil unterschiedlichsten weiteren Fachgebieten Verwendung, etwa bei operativen Eingriffen im Bauchraum, am Magen und zur Gallenblasenentfernung oder Blinddarmentfernung. Allerdings haben sich derartige Vorrichtungen in der Praxis zum Teil als verhältnismäßig nachteilig erwiesen. So besitzen derartige Vorrichtungen allesamt lediglich eine Schneideeinrichtung mit einem hohlzylindrischen Grundkörper, einer distalen Öffnung an einem distalen Ende des Grundkörpers und einem Schneideelement, welches die distale Öffnung umgibt. Der hohlzylindrische Grundkörper mit dem Schneideelement wird in Drehung versetzt, um den Gewebezylinder auszuschneiden bzw. auszustanzen. Aufgrund einer solchen Drehung des hohlzylindrischen Grundkörpers wird jedoch gleichzeitig das Gewebe, aus welchem der Gewebezylinder ausgeschnitten bzw. ausgestanzt werden soll, verformt bzw. bisweilen sogar in Eigenrotation versetzt. Um einen gezielten Eingriff an dem Gewebe vornehmen zu können, sind daher zusätzliche medizinische Instrumente, etwa Greifinstrumente, wie einem Krallengeifer, erforderlich, um das Gewebe entsprechend zu deformieren oder zu fixieren. Damit einhergehend gestaltet sich die Handhabung derartiger Vorrichtungen insgesamt aufwendig, für den Patienten wenig schonend und zeitintensiv.

Die Erfindung geht von der US-PS 4,306,570 als nächstkommenden Stand der Technik aus, die eine Vorrichtung laut dem Oberbegriff des Anspruchs 1 beschreibt.

Der Erfindung liegt daher die Aufgabe zugrunde, eine Vorrichtung zum Ausschneiden und Entfernen von Gewebezylindern aus einem Gewebe zur Verfügung zu stellen, wobei ein einfacher, schneller und damit kostengünstiger Austausch des Abtriebszahnrades beim Verschleiß oder sonstiger Reparatur gestattet ist.

Diese Aufgabe wird auf überraschend einfache Weise durch die Merkmale des kennzeichens des Anspruchs 1 gelöst.

Demnach lassen sich durch die Ausgestaltung der erfindungsgemäßen Vorrichtung nach Anspruch 1 eine einfache, kompakte und stabile Bauweise der gesamten Vorrichtung erreichen. Weiterhin hat die erfindungsgemäße Vorrichtung, die auch als Morcellator bezeichnet werden kann, den wesentlichen Vorteil, dass die Vorrichtung beim Ausschneiden bzw. Ausstanzen eines Gewebezylinders kein zusätzliches Instrument, insbesondere ein Greifinstrument, wie einen Krallengeifer oder dergleichen, zum Deformieren bzw. Halten des Gewebes während des operativen Eingriffs erfordert. Durch die Ausgestaltung der erfindungsgemäße Vorrichtung mit wenigstens zwei, insbesondere zwei, solcher Schneideelemente ist vielmehr eine Deformation bzw. Eigenrotation des Gewebes, aus welchem ein zu morcellierender Gewebezylinder ausgeschnitten bzw. ausgestanzt werden soll, ausgeschlossen. Nicht zuletzt hieraus resultierend ergibt sich eine ausgesprochen leichte, sehr schonende und vor allem zeitsparende Handhabung der Vorrichtung insgesamt. Schließlich lässt sich mit der Vorrichtung nach der Erfindung eine erheblich verbesserte und erhöhte Patientensicherheit erreichen.

Weitere vorteilhafte Einzelheiten der erfindungsgemäßen Vorrichtung sind in den Ansprüchen 2 bis 19 beschrieben.

So liegt es im Rahmen der Erfindung, dass die innere Schneideeinrichtung von der benachbarten äußeren Schneideeinrichtung der zwei Schneideeinrichtungen nach Anspruch 2 koaxial aufgenommen ist.

Zweckmäßigerweise ist die innere Schneideeinrichtung dabei von der benachbarten äußeren Schneideeinrichtung der zwei Schneideeinrichtungen mit geringem Spiel aufgenommen.

Alternativ ist die innere Schneideeinrichtung von der benachbarten äußeren Schneideeinrichtung der zwei Schneideeinrichtungen nach Anspruch 3 mit größerem Spiel aufgenommen, wobei wenigstens ein Zentrier- und/oder Führungselement zwischen den Grundkörpern der inneren Schneideeinrichtung und der benachbarten äußeren Schneideeinrichtung angeordnet ist/sind. Durch dieses Zentrier- und/oder Führungselement wird demnach eine koaxiale Ausrichtung der einander benachbarten Grundkörpers der Schneideeinrichtungen erreicht.

Weiterhin liegt es im Rahmen der Erfindung, dass die zwei Schneideeinrichtungen durch die Antriebseinrichtung zueinander im Gegenlauf und/oder im Gleichlauf antreibbar sind. Von ausgesprochen großer Bedeutung ist in diesem Zusammenhang vor allem die erfindungsgemäße Ausgestaltung eines gegenläufigen Antriebs der wenigstens zwei Schneideeinrichtungen durch die Antriebseinrichtung zueinander. Aufgrund eines Antriebs im Gegenlauf nämlich lässt sich noch zusätzlich erreichen, dass das Gewebe, aus welchem der Gewebezylinder ausgeschnitten bzw. ausgestanzt werden soll, keiner Verformung oder sogar Eigenrotation durch Beaufschlagung der Schneideeinrichtung unterzogen wird. Durch Drehung der wenigstens zwei, insbesondere zwei, Schneideeinrichtungen relativ zueinander heben sich die beim Ausschneiden bzw. Ausstanzen des Gewebezylinders auf das Gewebe einwirkenden Kräfte und Momente weitgehend gegeneinander auf.

Zu diesem Zweck ist das von einem Gehäuse aufgenommene Getriebe in vorteilhafter Weise zwischen den zwei Schneideeinrichtungen und der Antriebseinrichtung zwischengeordnet.

Zur konstruktiven Vereinfachung der erfindungsgemäßen Vorrichtung stehen die zwei Antriebszahnräder, die jeweils einer der zwei Schneideeinrichtungen zugeordnet sind, mit dem Abtriebszahnrad, das der Antriebseinrichtung zugeordnet ist, in gleichzeitigem Wirkeingriff. Die Drehachsen der zwei Antriebszahnräder fallen zusammen. Die Drehachsen der zwei Antriebszahnräder sind dagegen gegenüber der Drehachse des Abtriebszahnrades jeweils um ± 90° versetzt angeordnet. Mit einer solchen Ausbildung ist auf einfache Weise erreicht, dass die wenigstens zwei Schneideeinrichtungen zueinander im Gegenlauf antreibbar sind. Der Antrieb kann zudem nur durch eine einzige Antriebseinrichtung erfolgen.

Des Weiteren ist erfindungsgemäß vorgesehen, dass das Trägerelement des Abtriebszahnrades nach Anspruch 4 in vorteilhafter Weise an dem Gehäusedeckel des Gehäuses in einer Ausnehmung drehbar abgestützt ist. Durch eine solche zweiteilige Ausgestaltung des Abtriebszahnrades ist es möglich, das Trägerelement, das im Allgemeinen von der Antriebseinrichtung zur Übertragung der Drehbewegung mit nicht unerheblichen Kräften und/oder Momenten beaufschlagt wird, gegebenenfalls aus verschleißfesterem Material auszubilden. Auch ist aufgrund einer solchen zweiteiligen Ausgestaltung des Abtriebszahnrades ein einfacher, schneller und damit einhergehend kostengünstiger Austausch dessen bei Verschleiß möglich.

Zur Vereinfachung der Montage und/oder Reparatur sind das Abtriebszahnrad und das Trägerelement in diesem Zusammenhang nach Anspruch 5 in vorteilhafter Weise über eine Rast- oder Schnappverbindung oder Gewindeverbindung miteinander lösbar verbunden. Eine zusätzliche Sicherung kann durch Stifte etc. erfolgen.

Weiterhin liegt es im Rahmen der Erfindung, dass die zwei Antriebszahnräder jeweils von Antriebsbuchsen, die jeweils einer der zwei Schneideeinrichtungen zugeordnet sind, aufgenommen sind. Durch eine solche zweiteilige Ausgestaltung ist es ebenfalls möglich, unterschiedliches Material für die Antriebszahnräder und die Antriebsbuchsen entsprechend der jeweiligen Beanspruchung zu wählen. Auch ist damit ein Austausch zu Reparaturzwecken der Antriebsräder einfach, schnell und kostengünstig zu bewerkstelligen.

Zweckmäßigerweise sind die zwei Antriebszahnräder nach Anspruch 6 jeweils an den Antriebsbuchsen über eine Gewindeverbindung oder Rast- oder Schnappverbindung befestigt. Ohne im Einzelnen dargestellt zu sein, kann zusätzlich eine gesonderte Sicherung, beispielsweise mittels Stiften etc., vorgesehen sein.

Die Antriebsbuchsen ragen vorteilhafterweise durch das Gehäuse des Getriebes hindurch, um so eine konstruktive Anbindung zwischen den Antriebszahnrädern und den jeweils zugeordneten wenigstens zwei Schneideeinrichtungen zur Verfügung zu stellen.

Weiterhin liegt es im Rahmen der Erfindung, dass die Antriebseinrichtung nach Anspruch 7 mittels einer, insbesondere elektronischen, Steuer- und/oder Regeleinrichtung steuerbar und/oder regelbar ist, derart, dass die zwei Schneideeinrichtungen durch die Antriebseinrichtung jeweils intervallartig in entgegengesetzter Antriebsrichtung antreibbar sind. Hierdurch ist sichergestellt, dass zwei jeweils zueinander benachbarte Schneideeinrichtungen in beliebiger Weise in einer Rechts-Links-Drehung und/oder dazu reversibel und ebenso in beliebiger Weise in einer Links-Rechts-Drehung antreibbar sind.

In diesem Zusammenhang ist es von Vorteil, wenn die Zeitintervalle zum Antrieb der zwei Schneideeinrichtungen in entgegengesetzter Antriebsrichtung nach Anspruch 8 durch die, insbesondere elektronische, Steuer- und/oder Regeleinrichtung beliebig einstellbar und/oder voreinstellbar sind.

Die zwei Schneideeinrichtungen sind entsprechend den Merkmalen des Anspruchs 9 jeweils an dem proximalen Ende und/oder an dem dem proximalen Ende zugewandten Bereich des Grundkörpers mit Kupplungselementen versehen, die mit dem jeweiligen Grundkörper unlösbar verbunden sind.

Von besonderem Interesse sind die konstruktiven Maßnahmen des Anspruchs 10. Demnach sind die zwei Schneideeinrichtungen und die Antriebseinrichtung mit dem Getriebe jeweils über eine Kupplungseinrichtung drehfest verbindbar. Die Kupplungseinrichtung ist dabei in ihrer konstruktiven Ausbildung besonders einfach durch den jeweiligen Anwender handhabbar.

Zu diesem Zweck ist die Kupplungseinrichtung nach Anspruch 11 als Steck-, Rast- oder Schnappeinrichtung ausgebildet. Eine solche Steck-, Rast- oder Schnappeinrichtung ist besonders vorteilhaft, da während des operativen Eingriffs eine einfache und schnelle Anbindung der wenigstens zwei Schneideeinrichtungen und der Antriebseinrichtung mit dem Getriebe bzw. eine nachfolgende Trennung der Verbindung ermöglicht ist, und zwar unter Umständen sogar mit nur einer Hand des Anwenders.

In diesem Zusammenhang ist es besonders vorteilhaft, dass die Kupplungseinrichtung nach Anspruch 12 einen Kipphebel aufweist, der jeweils an den Antriebsbuchsen und dem Gehäuse des Getriebes um eine Drehachse verschwenkbar angeordnet ist, an einem Ende durch eine Feder beaufschlagt ist und an einem dem einen Ende gegenüberliegenden Ende einen etwa radial angeordneten Stift, Bolzen oder Vorsprung zum Eingreifen in einer korrespondieren ausgebildeten Bohrung oder Ausnehmung an dem Kupplungselement der wenigstens zwei Schneideeinrichtungen bzw. der Antriebseinrichtung umfasst.

Darüber hinaus liegt es im Rahmen der Erfindung, dass das Kupplungselement der inneren/innersten der zwei Schneideeinrichtungen nach Anspruch 13 als eine Dichtungseinrichtung zum Verschließen und Freigeben einer proximalen Öffnung an dem proximalen Ende des Grundkörpers ausgebildet ist.

In vorteilhafter Weise weist die Dichtungseinrichtung nach den Merkmalen des Anspruchs 14 ein zweigeteiltes und im Wesentlichen topfförmig ausgebildetes Gehäuse mit einem an dem Grundkörper angebrachten Gehäuseelement und einem an dem Gehäuseelement befestigbaren deckel- oder kappenförmiges Verschlusselement auf.

Zur einfachen, schnellen und zuverlässigen sowie vor allem kostengünstigen Herstellung und Montage sind das Gehäuseelement und das Verschlusselement des Gehäuses über eine Gewindeverbindung oder eine Rast- oder Schnappverbindung miteinander lösbar oder unlösbar verbunden. Die konstruktive Ausgestaltung, eine lösbare oder eine unlösbare Verbindung zwischen Gehäuseelement und Verschlusselement des Gehäuses vorzusehen, richtet sich dabei wesentlich nach der Verwendungs- und Bestimmungsart der erfindungsgemäßen Vorrichtung, d.h. ob die Vorrichtung zum Einmal- oder Mehrfach-Gebrauch vorgesehen sein soll. Dies wiederum, wie auch umgekehrt, wirkt sich erheblich auf die Ausbildung der Vorrichtung insgesamt bzw. die Auswahl der Materialien, aus welchen die einzelnen Komponenten der erfindungsgemäßen Vorrichtung bestehen, aus.

Ist die Vorrichtung beispielsweise lediglich für einen Einmal-Gebrauch bestimmt, wird also nach Gebrauch vollständig entsorgt, können das Gehäuseelement und das Verschlusselement des Gehäuses vorzugsweise unter Zwischenanordnung eines Klebemittels, insbesondere eines Silikonklebers, oder mittels Ultraschallverschweißens miteinander unlösbar verbunden sein.

Für den Mehrfach-Gebrauch werden das Gehäuseelement und das Verschlusselement des Gehäuses, die dann sicherlich auch aus Metall ausgebildet sind, hingegen mittels zum Beispiel eines Gewindes miteinander verschraubt.

Alternativ zur Gewindeverbindung kann eine Rast- oder Schnappverbindung vorgesehen sein, bei welcher das Gehäuseelement und das Verschlusselement des Gehäuses über wenigstens einen Rastvorsprung oder dergleichen Rastnase an dem Gehäuseelement und mindestens einen weiteren Rastvorsprung oder dergleichen Rastnase an dem Verschlusselement, die zusammenwirken und in gegenseitigen Eingriff bringbar sind, miteinander verbunden sind. Eine kinematische umkehr dessen, den wenigstens einen Rastvorsprung oder dergleichen Rastnase an dem Verschlusselement und die mindestens eine Rastausnehmung oder dergleichen an dem Gehäuseelement vorzusehen, ist ebenso möglich. Anstelle des mindestens einen weiteren Rastvorsprunges kann ebenso mindestens eine Rastausnehmung oder dergleichen vorgesehen sein, der mit dem wenigstens einen Rastvorsprung zusammenwirkt.

Für einen Einmal-Gebrauch ist die Vorrichtung nach der Erfindung in diesem Zusammenhang vorteilhafterweise derart ausgestaltet, dass der wenigstens eine Rastvorsprung oder dergleichen Rastnase an dem Gehäuseelement bzw. an dem Verschlusselement und der mindestens eine weitere Rastvorsprung oder dergleichen Rastnase an dem Verschlusselement bzw. an dem Gehäuseelement derart ausgebildet ist/sind, dass das Gehäuseelement und das Verschlusselement des Gehäuses entweder einerseits nach Ineingriffbringung von dem wenigstens einen Rastvorsprung oder dergleichen Rastnase und dem mindestens einen weiteren Rastvorsprung oder dergleichen Rastnase miteinander unlösbar verbunden sind oder andererseits nach anschließender Außereingriffbringung von dem wenigstens einen Rastvorsprung oder dergleichen Rastnase und dem mindestens einen weiteren Rastvorsprung oder dergleichen Rastnase miteinander unverbindbar sind. Bei letzterer Ausgestaltung wäre zum Beispiel denkbar, einen der beiden Rastvorsprünge oder dergleichen Rastnasen mit einer Sollbruchstelle zu versehen, so dass dieser Rastvorsprung oder dergleichen Rastnase bei Beaufschlagung einer bestimmten Zugkraft von dem Gehäuseelement bzw. von dem Verschlusselement abreißt. Anstelle des mindestens einen weiteren Rastvorsprunges kann ebenso mindestens eine Rastausnehmung oder dergleichen vorgesehen sein, der mit dem wenigstens einen Rastvorsprung zusammenwirkt.

Von ausgesprochen großer Bedeutung sind weiterhin die konstruktiven Maßnahmen des Anspruchs 14, wonach die Dichtungseinrichtung zwei Dichtungselemente umfasst, die in dem Gehäuse auf der Längsachse des Grundkörpers der Schneideeinrichtung zueinander benachbart angeordnet sind und miteinander zusammenwirken.

Dabei ist erfindungsgemäß vorgesehen, dass eines der zwei Dichtungselemente der Dichtungseinrichtung nach Anspruch 15 im Wesentlichen kegel-, kegelstumpf-, topf-, hut- oder dergleichen -förmig ausgebildet ist, eine Schlitzöffnung, die in einer Ebene, welche gegenüber einer durch die Grundfläche des Dichtungselementes aufgespannten Ebene in Richtung des distalen Endes des Grundkörpers der Schneideeinrichtung versetzt angeordnet ist, umfasst und einen sich radial nach außen erstreckenden Umfangsabschnitt in der durch die Grundfläche des Dichtungselementes aufgespannten Ebene zum Fixieren in dem Gehäuse aufweist. So wird das eine der zwei Dichtungselemente aufgrund seiner Formgebung und Anordnung in dem Gehäuse in entgegengesetzter Richtung zu der Wirkung eines innerhalb einer Körper- oder Gelenkhöhle künstlich aufgebauten Überdruckes, zum Beispiel durch CO₂-Insufflation, in dessen Schließstellung gezwungen und gehalten. Dabei wird das Dichtungselement an ein weiteres Instrument bzw. dessen Schaft, zum Beispiel ein Greif- oder sonstiges Schneideinstrument, insbesondere ein Krallengeifer oder ein Myombohrer, außenseitig unter vollständiger Abdichtung angedrückt. Demnach ist sichergestellt, dass der Überdruck, welcher zum Operieren, d.h. Ausschneiden bzw. Ausstanzen eines Gewebezylinders aus einem Gewebe und beim anschließenden Entfernen des ausgeschnittenen bzw. ausgestanzten Gewebezylinders aus dem Operationsgebiet erforderlich ist, in jedem Fall aufrechterhalten bleibt. Mithin ist ein Entweichen von Gas, wie CO₂ oder dergleichen, aus der Körper- oder Gelenkhöhle des Operationsgebietes ausgeschlossen und damit einer Behinderung des Aushülsungsvorganges oder einer Traumatisierung des umliegenden Gewebes bei der Aushülsung entgegenwirkt.

Insbesondere liegt es im Rahmen der Erfindung, dass das eine der zwei Dichtungselemente wenigstens zwei Dichtlippen aufweist, die zum Verschließen und Freigeben der Schlitzöffnung in gegenseitigem Wirkeingriff stehen.

Darüber hinaus ist ein anderes der zwei Dichtungselemente der Dichtungseinrichtung nach Anspruch 16 scheibenförmig ausgebildet und weist eine mittige, etwa kreisförmige Ausnehmung, durch welche ein weiteres Instrument, insbesondere ein Greif- oder sonstiges Schneideinstrument, vorzugsweise ein Krallengeifer oder ein Myombohrer, hindurchführbar ist, auf.

Die mittige, etwa kreisförmige Ausnehmung des anderen der zwei Dichtungselemente ist vorzugsweise mit einem Innendurchmesser ausgebildet, welcher kleiner oder gleich einem Außendurchmesser des weiteren Instrumentes ist.

Des Weiteren liegt es im Rahmen der Erfindung, dass das andere der zwei Dichtungselemente entsprechend konstruktiver Ausgestaltung in dem Gehäuse auf der Längsachse des Grundkörpers der Schneideeinrichtung zwischen dem proximalen Ende des Grundkörpers und dem einen der zwei Dichtungselemente, insbesondere der durch die Grundfläche des einen der zwei Dichtungselemente aufgespannten Ebene benachbart, angeordnet ist. Auf diese Weise wird ein gegenseitiges Zusammenwirken der beiden Dichtungselemente erreicht, wodurch die Abdichtung noch zusätzlich verbessert werden kann.

Zur Verbesserung der Dichtungswirkung ist/sind das wenigstens eine, insbesondere die zwei, Dichtungselement/e der Dichtungseinrichtung aus flexiblen Kunststoff, insbesondere aus Silikon, gebildet.

Von besonderem Interesse sind die Merkmale des Anspruchs 17. Danach sind die Schneideelemente der zwei Schneideeinrichtungen, welche die distale Öffnung umgeben, im Wesentlichen angefast, kegelförmig, konisch oder schräg von der distalen Öffnung abgewandt nach außen verlaufend und/oder zu der distalen Öffnung zugewandt nach innen verlaufend ausgebildet.

In diesem Zusammenhang weisen die Schneideelemente der zwei Schneideeinrichtungen nach Anspruch 18 vorteilhafterweise einen sehr scharfen, umlaufenden bzw. kontinuierlichen oder diskontinuierlichen Glattschliff, Wellenschliff oder Zahnschliff auf der bei einer Drehbewegung des Grundkörpers eine exakte Schnittführung in dem Gewebe bewirkt, ohne dass das verbleibende Gewebe zu stark traumatisiert wird. So lässt sich das Gewebe aushülsen, ohne dass umliegendes Gewebe bei dem Vorgang deformiert wird.

Die Schneideelemente der zwei Schneideeinrichtungen sind an dem vordersten Rand der distalen Öffnung bevorzugt mit einem Wellenschliff ausgestattet. Bei dieser Ausgestaltung der Erfindung ergeben sich mehrere, über den Umfang verteilte Kreisbogenabschnitte mit axial zurücktretenden Wellentälern und axial hervortretenden Wellenbergen. Beispielsweise würden bei vier Wellenvorsprüngen und vier dazwischen liegenden, leicht axial in der Umfangsfläche zurückgeführten Wellentälern Kreisbogenabschnitte etwa im Bereich von 40 bis 45° entstehen. Der Wellenschliff kann dabei umlaufend bzw. kontinuierlich, d.h. über den gesamten Umfang der distalen Öffnung, vorhanden sein. Zweckmäßigerweise kann die Schärfungszone im Übergangsbereich zwischen dem Wellental und dem Wellenvorsprung besonders scharf gestaltet sein, so dass zunächst eine axiale Fixierung des Gewebes und erst anschließend durch die Rotationsbewegung des Grundkörpers das Morcellieren von Gewebezylindern bzw. Gewebehülsen durchgeführt wird. Der Wellenschliff kann gegebenenfalls allerdings auch diskontinuierlich in Art einer schuppenförmigen Anordnung um den Mantelumfang an der distalen Öffnung vorgesehen sein.

Gleiches gilt für die alternative Ausgestaltung der Erfindung, wonach die Schneideelemente der zwei Schneideeinrichtungen mit dem Zahnschliff über den Umfang verteilte Zähne mit axial zurücktretenden Zahngründen und axial hervortretenden Zahnspitzen aufweisen. In Abhängigkeit von der gewünschten Verwendung ist ein umlaufender bzw. kontinuierlicher oder ein diskontinuierlicher Zahnschliff denkbar.

Zum Ausstanzen oder Aushülsen von Gewebe kann die Rotationsbewegung auf den Grundkörper über einen Motor, der elektrisch, mit Batterie, pneumatisch oder hydraulisch antreibbar ist, aufgebracht werden.

In besonders vorteilhafter Weise kann/können das Gehäuse des Getriebes und/oder das Zentrier- und/oder Führungselement und/oder das Abtriebszahnrad und/oder die zwei Antriebszahnräder und/oder das Trägerelement und/oder die Antriebsbuchsen und/oder die Kupplungselemente und/oder die Dichtungseinrichtung und/oder Gehäuseelement und/oder das Verschlusselement des Gehäuses und/oder die Klemmscheibe aus Kunststoff, insbesondere Polyoximethylen, Polyester, Polyphenylensulfon, ABS, Acryl, Polycarbonat, Tetrafluorethylen oder Impax, duroplastischen Elastomeren, mit oder ohne Glasfaserverstärkung, der einer Kombination hieraus gebildet sein. Die erfindungsgemäße Vorrichtung ist besonders leichtbauend und lässt sich ausgesprochen kostengünstig herstellen.

Alternativ dazu liegt es ebenso im Rahmen der Erfindung, dass die Grundkörper der zwei Schneideeinrichtungen und/oder das Gehäuse des Getriebes und/oder das Zentrier- und/oder Führungselement und/oder das Abtriebszahnrad und/oder das bzw. die zwei Antriebszahnräder und/oder das Trägerelement und/oder die Antriebsbuchsen und/oder die Kupplungselemente und/oder die Dichtungseinrichtung und/oder die Kupplungseinrichtung und/oder Gehäuseelement und/oder das Verschlusselement des Gehäuses und/oder die Klemmscheibe aus Metall, insbesondere (nicht-rostendem) Stahl, Edelstahl, Aluminium, Messing, Zink, Rotgusslegierungen oder einer Legierung hieraus gebildet ist/sind.

Die Vorrichtung lässt sich somit insgesamt thermisch und/oder chemisch sterilisieren und ohne weiteres für den Mehrfach-Gebrauch wiederverwenden.

Von besonderem Interesse sind weiter die konstruktiven Maßnahmen des Anspruchs 19. Demnach ist das Getriebe, das zwischen den zwei Schneideeinrichtungen und der Antriebseinrichtung angeordnet ist, mit dessen einzelnen Bauteilen in Form eines Baukastens ausgebildet und zusammenbaubar ist. Das Getriebe und damit die gesamte Vorrichtung lassen sich auf diese Weise baukastenähnlich vom Operateur oder dem Bedienungspersonal zusammensetzen. Einerseits können somit zusätzliche Montagekosten von Seiten des Herstellers eingespart werden. Andererseits lässt sich die erfindungsgemäße Vorrichtung dadurch einfach und schnell sowie zuverlässig, etwa zu Zwecken einer Desinfektion oder dergleichen, auseinanderbauen und anschließend bei Wiederverwendung erneut montieren.

In diesem Zusammenhang ist von großem Vorteil, wenn das Gehäuse des Getriebes bzw. das Gehäuseelement und der Gehäusedeckel und/oder das Abtriebszahnrad und/oder das bzw. die zwei Antriebszahnräder und/oder das Trägerelement in Form eines Baukastens ausgebildet und zusammenbaubar ist/sind.

Weitere Merkmale, Vorteile und Einzelheiten der Erfindung ergeben sich aus der nachfolgenden Beschreibung einer bevorzugten Ausführungsform der Erfindung sowie anhand der Zeichnungen. Hierbei zeigen:
- Fig. 1: eine perspektivische Vorderansicht einer Ausführungs- form einer erfindungsgemäßen Vorrichtung zum Aus- schneiden und Entfernen von Gewebezylindern aus einem Gewebe, ohne Gehäuse,
- Fig. 2: eine teilweise abgebrochene, perspektivische Ansicht der Ausführungsform der erfindungsgemäß ausgebildeten Vorrichtung entsprechend Ausschnitt II in der Fig. 1, in vergrößerter Darstellung,
- Fig. 3A bis 3F: eine Seitenansicht, eine Unteransicht, eine weitere Seitenansicht, eine Vorderansicht, eine Rück- ansicht und eine Draufsicht auf die Ausführungsform der erfindungsgemäß ausgebildeten Vorrichtung ent- sprechend den Fig. 1 und 2,
- Fig. 4: eine teilweise abgebrochene Seitenansicht der Ausfüh- rungsform der erfindungsgemäß ausgebildeten Vorrich- tung gemäß Fig. 3C, in vergrößerter Darstellung,
- Fig. 5A und 5B: eine Vorderansicht und eine Querschnittan- sicht durch die Ausführungsform der erfindungsgemäß ausgebildeten Vorrichtung längs der Linie VB-VB in der Fig. 5A, mit Gehäuse, in vergrößerter Darstel- lung,
- Fig. 6A und 6B: schematische Querschnittsansichten durch die Ausführungsform der erfindungsgemäß ausgebildeten Vorrichtung längs der Linie VIA-VIA in der Fig. 5B, in der Schließstellung und in der Offenstellung,
- Fig. 7A und 7B: schematische Querschnittsansichten durch die Ausführungsform der erfindungsgemäß ausgebildeten Vorrichtung längs der Linie VIIA-VIIA in der Fig. 5B, in der Schließstellung und in der Offenstellung, und
- Fig. 8: eine abgebrochene, schematische perspektivische An- sicht von Schneideelementen der Ausführung der erfin- dungsgemäß ausgebildeten Vorrichtung gemäß den Fig. 1 bis 7B, in vergrößerter Darstellung.

Die erfindungsgemäße Vorrichtung 10 ist zum Ausschneiden und Entfernen von Gewebezylindern aus einem Gewebe, das sich innerhalb einer Körper- oder Gelenkhöhle und/oder in bzw. an einem Wandbereich davon befindet, vorgesehen. Bei der nachfolgenden Beschreibung eines Ausführungsbeispieles der erfindungsgemäßen Vorrichtung 10 sind einander entsprechende, gleiche Bauteile jeweils mit identischen Bezugsziffern versehen.

Die Vorrichtung 10 nach der Erfindung eignet sich im weitesten Sinne zum Operieren, insbesondere Entfernen, eines organischen Gewebes, vorzugsweise von Myomen, Geschwülsten, Geschwüren, Karzinomen etc., oder eines anorganischen Körpers, wie Gallen- und Blasensteinen oder dergleichen Agglomerationen, die von einer Körper- oder Gelenkhöhle bzw. einem organischen Hohlraum eines menschlichen bzw. tierischen Körpers und/oder in bzw. an einem Wandbereich davon wenigstens teilweise aufgenommen und/oder umgeben sind.

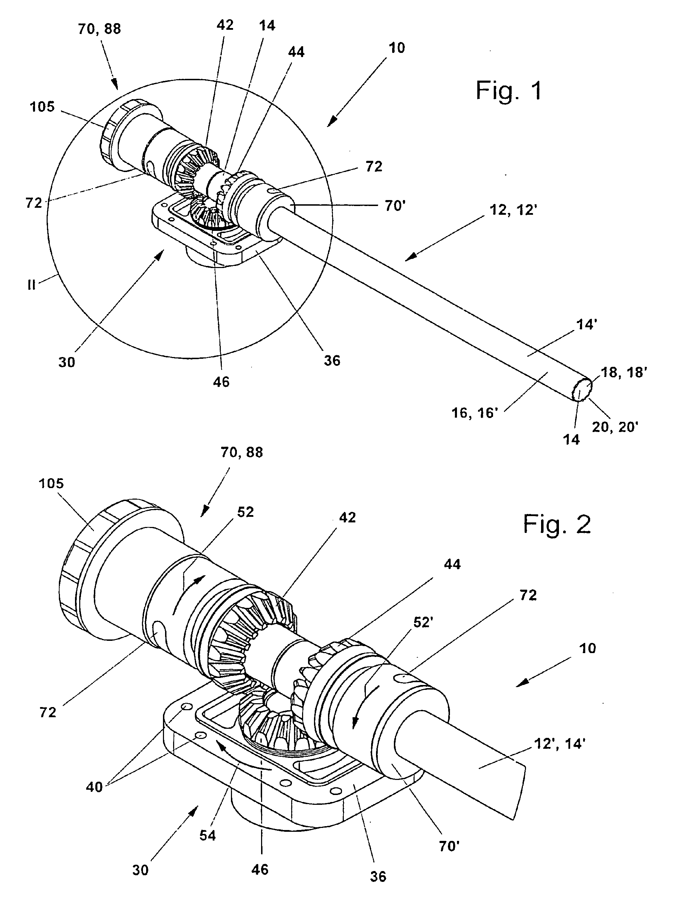

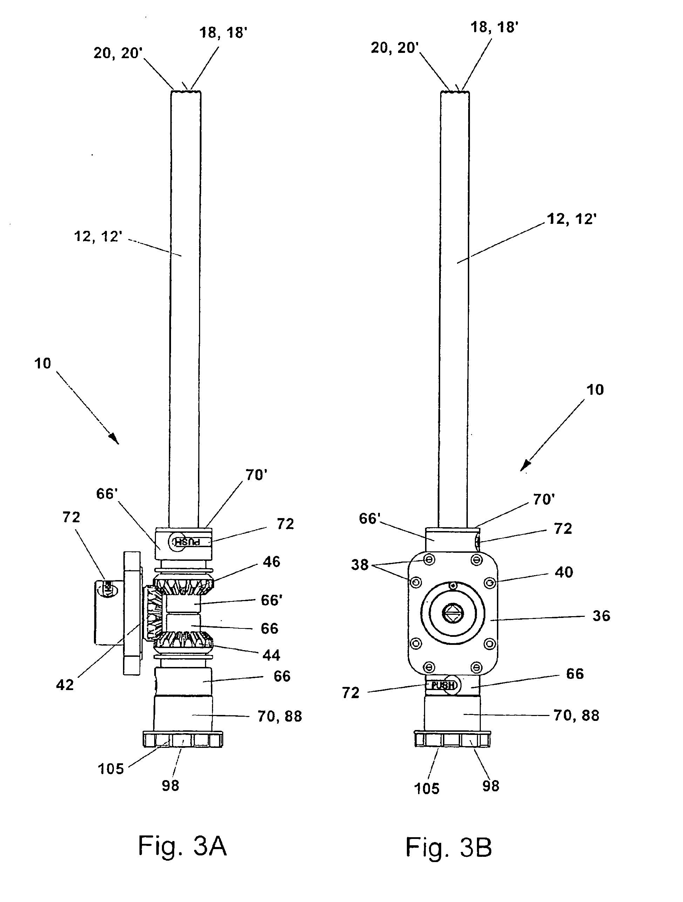

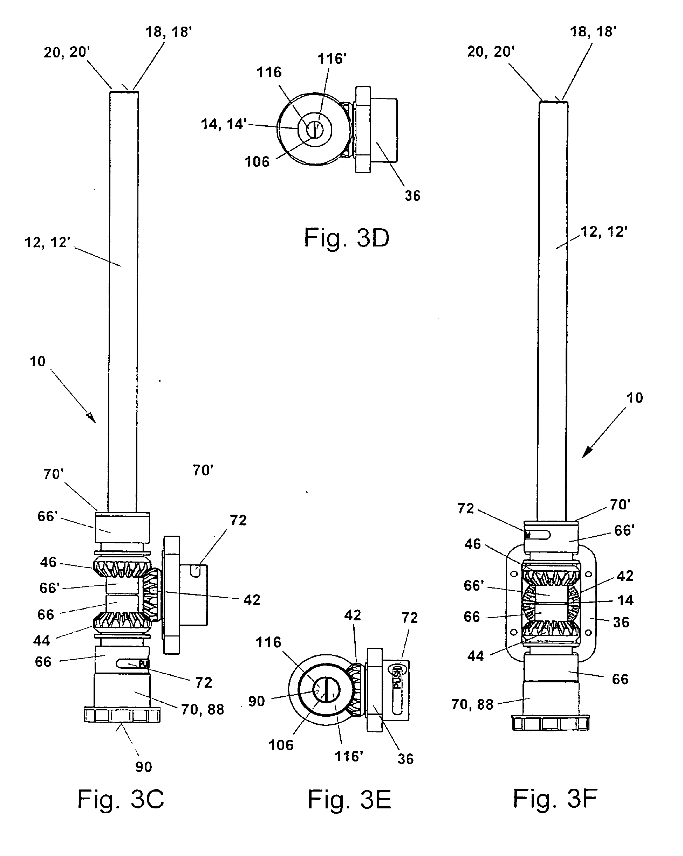

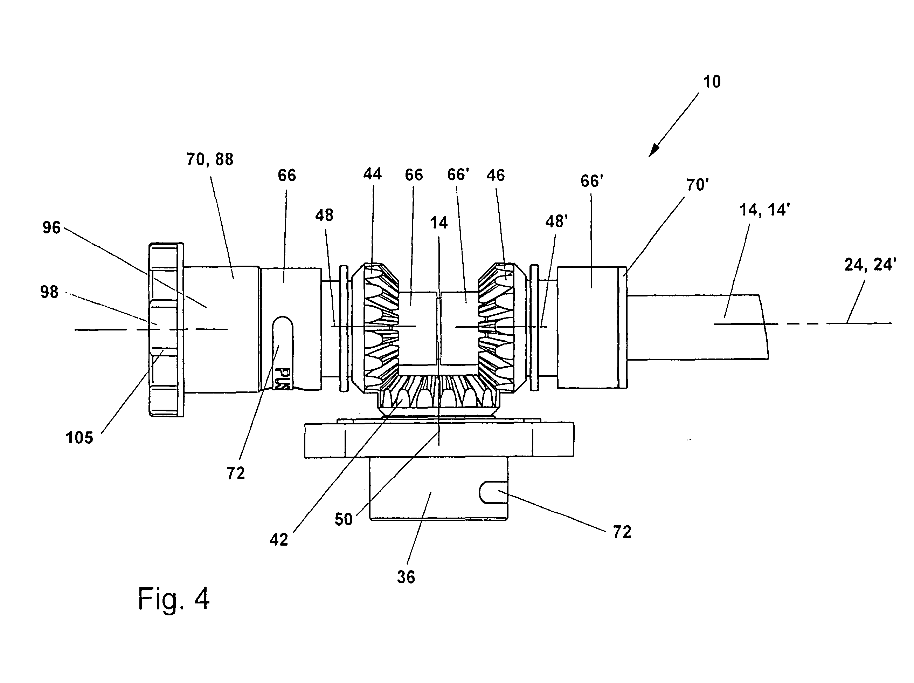

In den Fig. 1 bis 7B ist eine bevorzugte Ausführungsform einer solchen Vorrichtung 10 nach der Erfindung dargestellt.

Die Vorrichtung 10 umfasst wenigstens zwei Schneideeinrichtungen 12, 12' mit jeweils einem hohlzylindrischen Grundkörper 14, 14'. An dem distalen Ende 16, 16' des Grundkörpers 14, 14' befindet jeweils sich eine distale Öffnung 18, 18'. Die distale Öffnung 18, 18' ist jeweils von einem Schneideelement 20, 20' umgeben.

Bei der in den Fig. 1 bis 7B gezeigten Vorrichtung sind zwei solcher Schneideeinrichtungen 12, 12' vorgesehen, was einer besonders vorteilhaften Realisierung der Erfindung entspricht. Ohne im Einzelnen dargestellt zu sein, können jedoch grundsätzlich ebenso auch drei oder noch mehr Schneideeinrichtungen 12, 12' vorgesehen sein.

Die zwei Schneideeinrichtungen 12 und 12' sind dabei ineinander angeordnet. Insoweit ist das innere Schneideelement 20 von dem äußeren Schneideelement 20' aufgenommen. Weiterhin sind die zwei Schneideelemente 20, 20' zueinander relativ drehbar ausgebildet.

Die innere Schneideeinrichtung 12 ist somit von der benachbarten äußeren Schneideeinrichtung 12' der zwei Schneideeinrichtungen 12, 12' koaxial aufgenommen. Zwischen der inneren Schneideeinrichtung 12 und der benachbarten äußeren Schneideeinrichtung 12' ist vorzugsweise lediglich ein geringes Spiel vorhanden. Mithin ist der Außendurchmesser des Grundkörpers 14 der Schneideeinrichtung 12 geringfügig kleiner als der Innendurchmesser des Grundkörpers 14' der Schneideeinrichtung 12', und umgekehrt. Das Spiel zwischen der inneren Schneideeinrichtung 12 und der benachbarten äußeren Schneideeinrichtung 12' soll dabei derart ausgewählt sein, dass sich die Schneideeinrichtungen 12, 12' relativ zueinander leichtgängig drehen lassen, gleichzeitig aber eine exakte Führung zueinander bzw. untereinander zulassen. Zudem sollte ein Durchtritt von Blut, Körperflüssigkeit oder Gas etc. zwischen dem Außendurchmesser des Grundkörpers 14 der Schneideeinrichtung 12 und dem Innendurchmesser des Grundkörpers 14' der Schneideeinrichtung 12' möglichst bzw. weitgehend unterbunden sein.

Alternativ dazu wäre es möglich, ohne im Einzelnen dargestellt zu sein, dass die innere Schneideeinrichtung 12 von der benachbarten äußeren Schneideeinrichtung 12' auch mit größerem Spiel aufgenommen ist. Bei einer solchen Ausführungsform allerdings ist/sind ein oder mehrere Zentrier- und/oder Führungselemente (nicht dargestellt) zwischen den Grundkörpern 14, 14' der inneren Schneideeinrichtung 12 von der benachbarten äußeren Schneideeinrichtung 12' angeordnet. Diese/s Zentrier- und/oder Führungselement/e sollen insbesondere ein Verkanten und/oder eine Schwergängigkeit bei der Relativdrehung der inneren Schneideeinrichtung 12 gegenüber der benachbarten äußeren Schneideeinrichtung 12' sowie ein Durchtritt von Blut, Körperflüssigkeit oder Gas etc. verhindern.

Zum Drehen des jeweiligen Grundkörpers 14, 14' der beiden Schneideneinrichtungen 12, 12' um deren jeweilige Längsachse 24, 24' ist die Vorrichtung 10' mit mindestens einer Antriebseinrichtung 22 versehen. Beim vorliegenden Ausführungsbeispiel der Vorrichtung 10 ist lediglich eine solche Antriebseinrichtung 22 vorgesehen (schematisch dargestellt). Die Antriebseinrichtung 22 kann an einem proximalen Ende 26 eines der Grundkörper 14, 14' angeordnet sein. Alternativ oder - wie bei dem vorliegenden Ausführungsbeispiel gezeigt ist - ist die Antriebseinrichtung 22 auch an einem Bereich 28 des Grundkörpers 14 angeordnet, welcher dem proximalen Ende 26 lediglich zugewandt und/oder benachbart ist. Die Antriebseinrichtung 22 ist als Motor ausgebildet, der beispielsweise elektrisch, mit Batterie, pneumatisch oder hydraulisch antreibbar ist.

In ganz besonders vorteilhafter Weise sind die zwei Schneideeinrichtungen 12, 12' durch die Antriebseinrichtung 22 zueinander im Gegenlauf antreibbar. Hierdurch lässt sich zusätzlich auf ganz besonders einfache und damit auch kostengünstige Weise erreichen, dass das Gewebe (nicht gezeigt), aus welchem der entsprechende Gewebezylinder ausgeschnitten und/oder ausgestanzt werden soll, bei dem operativen Eingriff mittels der erfindungsgemäßen Vorrichtung 10 innerhalb der Körper- oder Gelenkhöhle und/oder in bzw. an dem Wandbereich davon weder deformiert noch in Eigenrotation verbracht wird. Durch die gegenläufige Drehung der zwei Schneideeinrichtungen 12, 12' werden jedenfalls die durch die entsprechenden Schneideelemente 20, 20' auf das Gewebe einwirkenden Kräfte und/oder eventuellen Momente gegeneinander aufgehoben. Die Folge davon ist, dass das Gewebe während des operativen Eingriffs durch die Vorrichtung 10 nach der Erfindung undeformiert bzw. in einer bestimmten Lage ohne Eigenbewegung verbleibt.

Ohne im Einzelnen dargestellt zu sein, kann die Antriebseinrichtung 22 mittels einer, insbesondere elektronischen, Steuer- und/oder Regeleinrichtung (nicht dargestellt) steuerbar und/oder regelbar sein, so dass die zwei Schneideeinrichtungen 12, 12' durch die Antriebseinrichtung 22 jeweils intervallartig in entgegengesetzter Antriebsrichtung antreibbar sind. Die, etwa oszillierende oder dergleichen wechselnde, Änderung der Antriebsrichtung der zwei Schneideeinrichtungen 12, 12' ist unabhängig von deren relativen Drehbewegung zueinander. Mit anderen Worten kann ein Antrieb der zwei Schneideeinrichtungen 12, 12' in entgegengesetzter Antriebsrichtung erfolgen, ohne dass deren relativer Antrieb zueinander im Gegenlauf und/oder im Gleichlauf aufgehoben wird. Hierdurch ist sichergestellt, dass zwei jeweils zueinander benachbarte Schneideeinrichtungen 12, 12' in beliebiger Weise in einer Rechts-Links-Drehung und/oder dazu reversibel und ebenso in beliebiger Weise in einer Links-Rechts-Drehung antreibbar sind.

Dabei ist es von besonderem Vorteil, wenn die Zeitintervalle zum Antrieb der zwei Schneideeinrichtungen 12, 12' in entgegengesetzter Antriebsrichtung durch die, insbesondere elektronische, Steuer- und/oder Regeleinrichtung beliebig einstellbar und/oder voreinstellbar sind. Die Zeitintervalle können in diesem Zusammenhang gleich oder unterschiedlich lang gewählt werden, genau an den jeweils individuell anzutreffenden Operationsbereich angepasst oder stochastisch gebildet werden, etc.

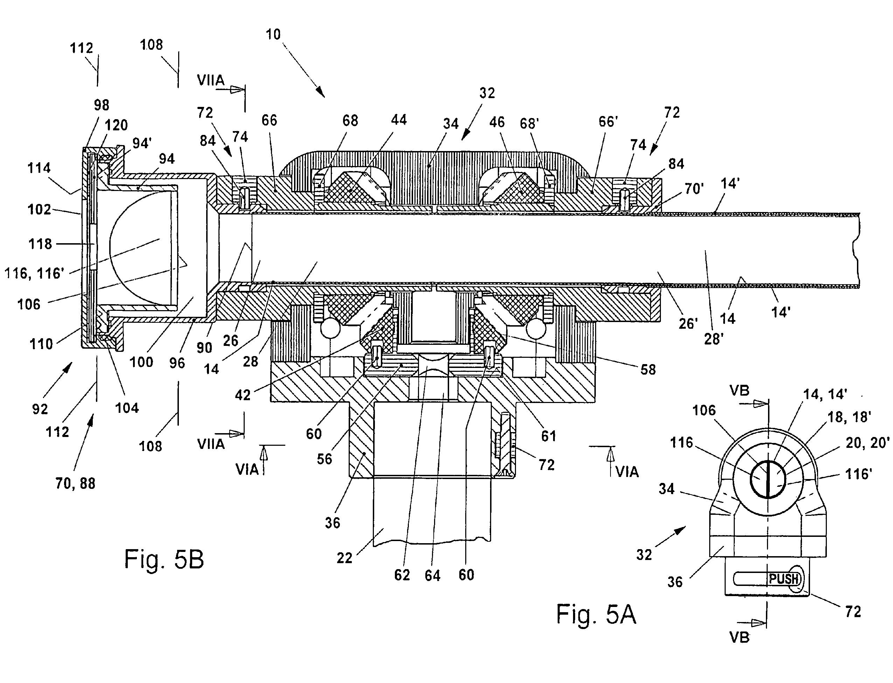

Um einen Antrieb der zwei Schneideeinrichtungen 12, 12' durch die Antriebseinrichtung 22 zueinander im Gegenlauf und/oder im Gleichlauf zu erreichen, ist zwischen den zwei Schneideeinrichtungen 12, 12' und der Antriebseinrichtung 22 ein Getriebe 30 zwischengeordnet, das in einem Gehäuse 32 untergebracht ist. Das Gehäuse 32 besteht dabei aus einem Gehäuseelement 34, welches lediglich in den Fig. 5A und 5B gezeigt ist, und einem Gehäusedeckel 36, welcher sämtlichen Fig. 1 bis 7B entnehmbar ist. Das Gehäuseelement 34 und der Gehäusedeckel 36 sind miteinander über Schrauben 38, welche entsprechende Bohrungen bzw. Ausnehmungen in dem Gehäuseelement 34 durchgreifen (nicht dargestellt) und in entsprechenden Bohrungen 40 bzw. Ausnehmungen in dem Gehäusedeckel 36 in herkömmlicher Weise konterbar sind.

Das Getriebe 30 umfasst zum Antrieb im Gegenlauf ein Abtriebszahnrad 42, welches der Antriebseinrichtung 22 zugeordnet ist. Darüber hinaus weist das Getriebe 30 zwei Antriebszahnräder 44, 46 auf, die mit dem Abtriebszahnrad 42 in Wirkeingriff stehen und jeweils einem der zwei Schneideeinrichtungen 12, 12' zugeordnet sind.

Das Abtriebszahnrad 42 und die zwei Antriebszahnräder 44, 46 des Getriebes 30 sind bei der in den Fig. 1 bis 7B dargestellten Ausführungsform der erfindungsgemäßen Vorrichtung 10 jeweils als Kegelräder ausgebildet.

Bei der in den Fig. 1 bis 7B gezeigten Ausführungsform der Vorrichtung 10 stehen die zwei Antriebszahnräder 44 bzw. 46, die jeweils einer der zwei Schneideeinrichtungen 12 bzw. 12' zugeordnet sind, mit dem Abtriebszahnrad 42, das der Antriebseinrichtung 22 zugeordnet ist, in gleichzeitigem Wirkeingriff. Bei der vorliegenden Ausführungsform fallen die Drehachsen 48, 48' der zwei Antriebszahnräder 44, 46 zusammen. Die Drehachsen 48, 48' der zwei Antriebsräder 44, 46 sind zudem identisch mit den Längsachsen 24, 24' der Grundkörper 14, 14'. Demgegenüber sind die Drehachsen 48, 98' der zwei Antriebszahnräder 44, 46 gegenüber der Drehachse 50 des Abtriebszähnrades 42 einmal um + 90° und einmal um - 90° versetzt angeordnet. Durch eine solche konstruktive Ausgestaltung ist eine gleichzeitige, gleichförmige, zudem aber entgegengesetzte Drehung der zwei Antriebszahnräder 44, 46 entsprechend den Pfeilen 52, 52' durch Drehung des Abtriebszahnrades 42 entsprechend Pfeil 54 erreicht. Dreht das Abtriebszahnrad 42 hingegen in zu dem Pfeil 54 entgegengesetzter Richtung kehrt sich auch die Drehrichtung der beiden Antriebszahnräder 44, 46 entsprechend den Pfeilen 52, 52' um (nicht dargestellt).

Wie insbesondere aus den Fig. 2 und 5B hervorgeht, ist das Abtriebszahnrad 42 weiterhin an einem etwa scheibenförmigen Trägerelement 56 lösbar befestigt. Zu diesem Zweck sind das Abtriebszahnrad 44 und das Trägerelement 56 über eine Rast- oder Schnappverbindung 58 miteinander lösbar verbunden. Zusätzlich sind Stifte 60 bzw. Bolzen oder dergleichen vorgesehen., um eine Relativbewegung zwischen Abtriebszahnrad 42 und Trägerelement 56 zu verhindern.

Das scheibenförmige Trägerelement 56 ist an dem Gehäusedeckel 36 des Gehäuses 32 von dem Getriebe 30 drehbar abgestützt. Beispielsweise ist das Trägerelement 5.6 in einer runden Ausnehmung 61 eingebracht. Das Trägerelement 56 ist weiterhin mit einer mittig angeordneten Ausnehmung 62 versehen, in welcher ein zapfenförmiges Übertragungselement 64 des Antriebseinrichtung 22 drehfest eingreift. Die mittig angeordnete Ausnehmung 62 und das Übertragungselement 64 sind einander korrespondierend ausgebildet. So kann das Übertragungselement 64 beispielsweise mit einem im Querschnitt rechteckförmigen, quadratischen sechseckförmigen oder sonstwie polygonalen, elliptischen, trapezoiden, parallelogrammartigen oder halbkreisförmigen Profil ausgestattet sein.

Die zweiteilige konstruktive Ausbildung von Abtriebszahnrad 42 und Trägerelement 56 gestattet zum einen den Einsatz von verschiedenartigen Materialien mit unterschiedlichen Eigenschaften. Beispielsweise ist das Abtriebszahnrad 42 bei dem vorliegenden Ausführungsbeispiel der Vorrichtung 10 aus Polyoximethylen (POM) gebildet, während das Trägerelement 56 aus Polyphenylensulfon (PPSU) besteht. Bekanntlich ist Polyphenylensulfon beim Auftreten hoher Kräfte und/oder Momente, die von der Antriebseinrichtung 22 über das Übertragungselement 64 auf das Trägerelement 56 einwirken, weitaus widerstandsfähiger und verschleißfester als mancher anderer Kunststoff. Zum anderen gestattet eine solche konstruktive Ausbildung einen einfachen, schnellen und damit kostengünstigen Austausch des Abtriebszahnrades 42 beim Verschleiß oder sonstiger Reparatur.

Gleiches gilt für die zwei Antriebszahnräder 44, 46, welche bei der vorliegenden Ausführungsform der Vorrichtung 10, die in den Fig. 1 bis 7B gezeigt ist, ebenfalls zweiteilig ausgestaltet sind. Demnach sind die zwei Antriebszahnräder 44, 46 jeweils von Antriebsbuchsen 66, 66' aufgenommen, welche jeweils einer der zwei Schneideeinrichtungen 12, 12' zugeordnet sind.

Entsprechend der Fig. 5B sind die zwei Antriebszahnräder 44, 46 bei der dargestellten Vorrichtung 10 jeweils an den Antriebsbuchsen 66, 66' über eine Gewindeverbindung befestigt. Zur Sicherung sind (Unterleg- bzw. Anlauf-)Scheiben 68, 68' vorgesehen, an welchen die Antriebszahnräder 44, 46 konterbar sind. Zusätzlich ist es denkbar, die Antriebszahnräder 44, 46 mittels Stiften oder Bolzen (nicht gezeigt) bzw. unter Zwischenordnung eines Klebemittels, vorzugsweise eines Silikonklebers, oder mittels Ultraschallverschweißens zu sichern, gleichwohl eine solche zusätzliche Sicherung eine schnelle Montage bzw. Demontage zu Reparaturzwecken erschweren könnte. Die Antriebsbuchsen 66, 66' erstrecken sich, wie in der Fig. 5B deutlich erkennbar ist, selbst durch das Gehäuse 32 des Getriebes 30 hindurch.

Die wenigstens zwei Schneideeinrichtungen 12, 12', wie ebenfalls der Fig. 5B entnehmbar ist, sind mit Kupplungselementen 70, 70' versehen, die mit dem jeweiligen Grundkörper 14, 14' der Schneideeinrichtungen 12, 12' unlösbar verbunden sind. Die Kupplungselemente 70, 70' können dabei an dem proximalen Ende 26, 26' angeordnet sein. Alternativ oder kumulativ sind die Kupplungselemente 70, 70', wie bei dem dargestellten Ausführungsbeispiel, in einem Bereich 28, 28' des Grundkörpers 14, 14' angeordnet, welche dem proximalen Ende 26, 26' zugewandt ist.

Die zwei Schneideeinrichtungen 12, 12' und die Antriebseinrichtung 22 sind des Weiteren mit dem Getriebe 30 jeweils über eine Kupplungseinrichtung 72 drehfest verbindbar. Die Kupplungseinrichtung 72 ist als Steck-, Rast- oder Schnappeinrichtung ausgebildet.

Die Kupplungseinrichtung 72 weist zu diesem Zweck einen Kipphebel 74 auf. Der Kipphebel 74 ist jeweils an den Antriebsbuchsen 66, 66' und dem Gehäuse 32 des Getriebes 30 um eine Drehachse 76 verschwenkbar angeordnet. An einem Ende 78 ist der Kipphebel 74 durch eine Feder 80 beaufschlagt. An einem Ende 82, welches dem Ende 78 gegenüberliegt, ist ein Stift 84, Bolzen oder Vorsprung vorgesehen, der mit einer korrespondierend ausgebildeten Bohrung 86 oder Ausnehmung an dem zugeordneten Kupplungselement 70, 70' der zwei Schneideeinrichtung 12, 12' bzw. der Antriebseinrichtung 22 zusammenwirkt (nicht dargestellt). Der Stift 84, Bolzen oder Vorsprung und die dazugehörige Bohrung 86 oder Ausnehmung verlaufen jeweils etwa radial zu den Längsachsen 24, 24' der Grundkörper 14, 14' der Vorrichtung 10.

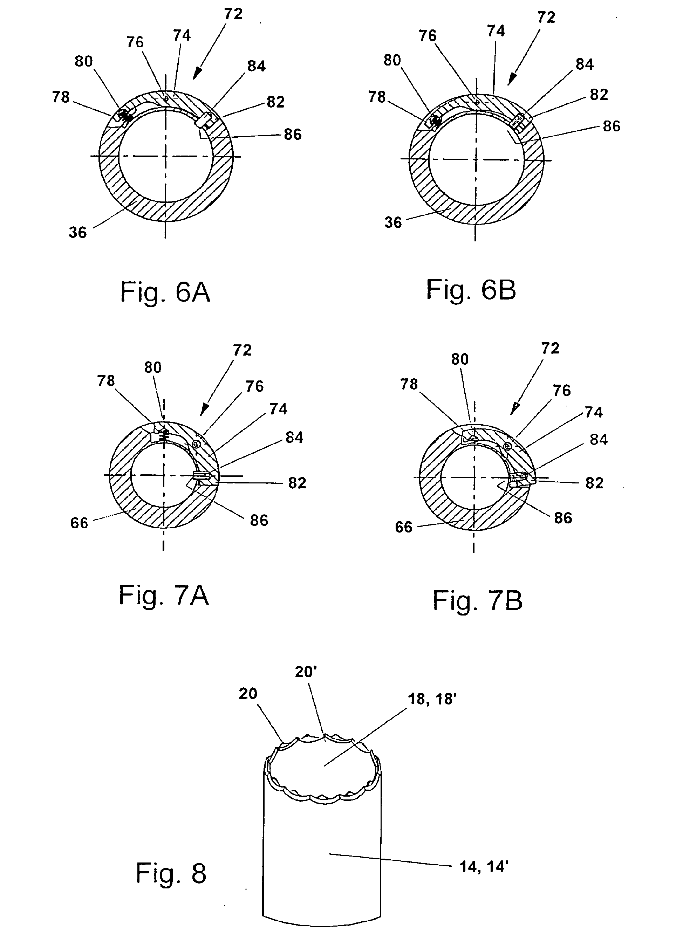

Die Feder 80 ist derart angeordnet, dass der Kipphebel 74 ständig in der Schließstellung der Kupplungseinrichtung 72 gehalten ist. In der Schließstellung, welche in den Fig. 6A und 7A dargestellt ist, befindet sich der radial verlaufende Stift 84, Bolzen oder Vorsprung mit der Bohrung 86 oder Ausnehmung in Eingriff. Die jeweilige Schneideeinrichtung 12, 12' bzw. die Antriebseinrichtung 22 sind somit drehfest an das Getriebe 30 angebunden.

In der Offenstellung der Kupplungseinrichtung 72 hingegen, welche durch Herunterdrücken des Kipphebels 64 entgegen der Kraftwirkung der Feder 80 erreicht wird, wird der Kipphebel 74 mit dem Stift 84, Bolzen oder Vorsprung radial nach außen verschwenkt. Der Stift 84, Bolzen oder Vorsprung kommt infolge dessen mit der Bohrung 86 oder Ausnehmung in der Offenstellung, die in den Fig. 6B und 7B gezeigt ist, außer Eingriff. Die Schneideeinrichtungen 12, 12' bzw. die Antriebseinrichtung 22 lassen sich somit einfach von dem Getriebe 30 trennen.

Bei der Ausführung der Vorrichtung 10 nach der Erfindung, die in den Fig. 1 bis 7B gezeigt ist, ist das Kupplungselement 70, das der inneren Schneideeinrichtung 12 der zwei Schneideeinrichtungen 12, 12' zugeordnet ist, als eine Dichtungseinrichtung 88 ausgebildet.

Die Dichtungseinrichtung 88 dient zum Verschließen und Freigeben einer proximalen Öffnung 90, die sich an dem proximalen Ende 26 des Grundkörpers 14 befindet. Die Dichtungseinrichtung 88 umfasst ein Gehäuse 92 und wenigstens ein in dem Gehäuse 92 aufnehmbares Dichtungselement 94, 94'. Wie aus den Fig. 1 bis 7B ersichtlich ist, sind bei der gezeigten Ausführungsform der erfindungsgemäßen Vorrichtung 10 insgesamt zwei Dichtungselemente 94, 94' vorgesehen. Ohne im Einzelnen dargestellt zu sein, ist es ohne weiteres denkbar, lediglich ein einziges Dichtungselement, zum Beispiel als integrales Bauteil der beiden gezeigten Dichtungselemente 94, 94', oder ebenso mehr als zwei solcher Dichtungselemente 94, 94' vorzusehen.

Das Gehäuse 92 ist aus einem Gehäuseelement 96 und einem Verschlusselement 98 gebildet.

Das Gehäuseelement 96 ist im Wesentlichen topfförmig ausgebildet, nimmt das wenigstens eine Dichtungselement 94, 94' auf und ist an dem Grundkörper 14 unlösbar angebracht. Das wenigstens eine Dichtungselement 94, 94' erstreckt sich zumindest zum Teil in einer Kammer 100, die von dem Gehäuseelement 96 des Gehäuses 92 eingeschlossen und somit gebildet ist.

Das Verschlusselement 98 ist deckel- oder kappenförmig ausgestaltet. Das Verschlusselement 98 ist mit einer mittigen, etwa kreisförmig ausgestalteten Bohrung 102 oder dergleichen Ausnehmung versehen. Durch die Bohrung 102 ist ein weiteres Instrument (nicht gezeigt), insbesondere ein Greif- oder sonstiges Schneideinstrument, vorzugsweise ein Krallengeifer oder ein Myombohrer, hindurchführbar, nämlich über die Bohrung 102 durch die proximale Öffnung 90 des Grundkörpers 14, durch die Dichtungseinrichtung 88 am proximalen Ende 26 des Grundkörpers 14, durch den Grundkörper 14 selbst hin zu dem distalen Ende 16 des Grundkörpers 14, durch die distale Öffnung 18 schließlich zu dem zu entfernenden Gewebe(-zylinder) in dem Operationsgebiet. Das Verschlusselement 98 ist wiederum an dem Gehäuseelement 96 befestigbar.

Zu diesem Zweck können das Gehäuseelement 96 und das Verschlusselement 98 des Gehäuses 92 über eine Gewindeverbindung bzw. Rast- oder Schnappverbindung 104 miteinander verbunden sein, und zwar entweder lösbar oder unlösbar. Die konstruktive Ausgestaltung einer lösbaren oder einer unlösbaren Verbindung zwischen Gehäuseelement 96 und Verschlusselement 98 ist abhängig von der Verwendungs- und Bestimmungsart der Vorrichtung 10 in Form eines Einmal- oder Mehrfach-Gebrauchs, der Ausbildung der Vorrichtung 10 bzw. der Auswahl der Materialien, aus welchen die einzelnen Komponenten der erfindungsgemäßen Vorrichtung 10 bestehen, den Wünschen der Anwender usw.

Bei einem Einmal-Gebrauch der Vorrichtung 10 sind das Gehäuseelement 96 und das Verschlusselement 98 über die Gewindeverbindung bzw. Rast- oder Schnappverbindung 104 unter Zwischenanordnung eines Klebemittels, vorzugsweise eines Silikonklebers, oder mittels Ultraschallverschweißens miteinander unlösbar verbunden. Auf diese Weise sind eine thermische oder chemische Sterilisation de Vorrichtung 10 und jeder nochmalige bzw. weitere Gebrauch der Vorrichtung 10 ausgeschlossen.

Das Gehäuse 92 der Dichtungseinrichtung 88 und insbesondere das Verschlusselement 98 des Gehäuses 92, ist an dessen Umfang mit einem Profil 105 zum manuellen Betätigen und Drehen des Grundkörpers 14 versehen.

Die Dichtungseinrichtung 88 umfasst, wie bereits erwähnt, bei der Ausführung der Fig. 1 bis 7B zwei Dichtungselemente 94, 94', die in dem Gehäuse 92 auf der Längsachse 24 des Grundkörpers 14 der Schneideeinrichtung 12 zueinander benachbart angeordnet sind und miteinander zusammenwirken.

Eines 94 der zwei Dichtungselemente 94, 94' der Dichtungseinrichtung 88 ist im Wesentlichen kegel-, kegelstumpf-, topf-, hut- oder dergleichen -förmig ausgebildet. Das Dichtungselement 94 umfasst eine Schlitzöffnung 106, die in einer Ebene 108 (senkrecht zur Blattebene), welche gegenüber einer durch die Grundfläche 110 des Dichtungselementes 94 aufgespannten Ebene 112 (senkrecht zur Blattebene) in Richtung des distalen Endes 16 des Grundkörpers 14 versetzt angeordnet ist. Das Dichtungselement 94 erstreckt sich daher mit dem im Wesentlichen kegel-, kegelstumpf-, topf-, hut- oder dergleichen -förmig ausgebildeten Teil bzw. Abschnitt in Richtung des distalen Endes 16 des Grundkörpers 14 und ragt in die Kammer 100 des Gehäuses 92. Zudem weist das Dichtungselement 94 einen sich radial nach außen erstreckenden Umfangsabschnitt 114 auf, der in der durch die Grundfläche 110 des Dichtungselementes 94 aufgespannten Ebene 112 liegt und einer Fixierung in dem Gehäuse 92 dient.

Das Dichtungselement 94 weist wenigstens zwei Dichtlippen 116, 116' auf, die zum Verschließen und Freigeben der Schlitzöffnung 106 in gegenseitigem Wirkeingriff stehen. Aufgrund der Formgebung und Anordnung der zwei Dichtlippen 116, 116' des Dichtungselementes 94 in dem Gehäuse 92 in entgegengesetzter Richtung zu der Wirkung eines innerhalb einer Körper- oder Gelenkhöhle mittels zum Beispiel durch CO₂-Insufflation künstlich aufgebauten Überdruckes, schmiegen sich die Dichtlippen 116, 116' an ein weiteres Instrument (nicht gezeigt), zum Beispiel ein Greif- oder sonstiges Schneideinstrument, insbesondere ein Krallengeifer oder ein Myombohrer, außenseitig an, und zwar unter vollständiger Abdichtung. Auf diese Weise findet quasi eine Verkeilung der Dichtlippen 116, 116' statt. Die Dichtlippen 116, 116' werden an das weitere Instrument angedrückt und verbleiben in dieser Stellung, bis der Überdruck geregelt aufgehoben wird. Somit ist gewährleistete, dass der Überdruck, welcher zum Operieren, d.h. Ausschneiden bzw. Ausstanzen eines Gewebezylinders aus einem Gewebe und beim anschließenden Entfernen des ausgeschnittenen bzw. ausgestanzten Gewebezyliriders aus dem Operationsgebiet notwendig ist, dauerhaft aufrechterhalten bleibt. Ein Entweichen von Gas, wie CO₂ oder dergleichen, aus der Körper- oder Gelenkhöhle des Operationsgebietes ist wirksam verhindert.

Ein anderes 94' der zwei Dichtungselemente 94, 94' der Dichtungseinrichtung 88 ist scheibenförmig ausgebildet und ist mit einer mittigen, etwa kreisförmigen Ausnehmung 118 versehen. Durch die Ausnehmung 118 des Dichtungselementes 94' ist das bereits erwähnte, weitere Instrument (nicht gezeigt), insbesondere ein Greif- oder sonstiges Schneideinstrument, vorzugsweise ein Krallengeifer oder ein Myombohrer, hindurchführbar. Die mittige, etwa kreisförmige Ausnehmung 118 ist mit einem Innendurchmesser, welcher kleiner oder gleich einem Außendurchmesser des weiteren Instrumentes ist, versehen.

Wie aus der Fig. 5B ersichtlich ist, ist das andere Dichtungselement 94' in dem Gehäuse, 92 auf der Längsachse 24 des Grundkörpers 14 zwischen dem proximalen Ende 26 des Grundkörpers 14 und dem einen Dichtungselement 94 angeordnet. Insbesondere ist das andere Dichtungselement 94' der Ebene 112, welche durch die Grundfläche 110 des einen Dichtungselementes 94 aufgespannt ist, (unmittelbar) benachbart zugeordnet.

Die zwei Dichtungselemente 94, 94' der Dichtungseinrichtung 88 sind durch eine Klemmscheibe 120 oder dergleichen in dem Gehäuse 92 zueinander fixierbar.

Entsprechend den Fig. 1 und 8 sind die Schneideelemente 20, 20' der Schneideeinrichtungen 12, 12' im Wesentlichen angefast, kegelförmig, konisch oder schräg von der distalen Öffnung 18, 18' abgewandt nach außen und/oder zu der distalen Öffnung 18 zugewandt nach innen verlaufend ausgebildet. Bei der Ausführung der Vorrichtung 10, die in der Fig. 8 gezeigt ist, ist das Schneideelement 20 der Schneideeinrichtung 12 im Wesentlichen angefast, kegelförmig, konisch öder schräg zu der distalen Öffnung 18 zugewandt nach innen verlaufend ausgebildet. Demgegenüber ist das Schneideelement 20' der Schneideeinrichtung 12' im Wesentlichen angefast, kegelförmig, konisch oder schräg von der distalen Öffnung 18' abgewandt nach außen verlaufend angeordnet. Ohne im Einzelnen dargestellt zu sein, sind beliebige andere konstruktive Ausgestaltungen bzw. Anordnungen der Schneideelemente 20, 20' zueinander ebenso möglich.

Dabei können die Schneideelemente 20, 20' jeweils einen umlaufenden oder diskontinuierlichen Glattschliff, Wellenschliff oder Zahnschliff aufweisen. Bei der Ausführung der Vorrichtung 10, wie vor allem aus der Fig. 8 hervorgeht, sind beide Schneideelemente 20, 20' mit einem Zahnschliff versehen, der über den Umfang verteilte Zähne mit axial zurücktretenden Zahngründen und axial hervortretenden Zahnspitzen aufweist. Ohne im Einzelnen dargestellt zu sein, sind auch diesbezüglich beliebige andere Kombinationen realisierbar. So könnten die Schneideelemente 20, 20' der zwei Schneideeinrichtungen 12, 12' zum Beispiel im Wellenschliff ausgestaltet sein, der über den Umfang verteilte Kreisbogenabschnitte mit axial zurücktretenden Wellentälern und axial hervortretenden Wellenbergen aufweist.

Das wenigstens eine, insbesondere die zwei, Dichtungselement/e 94, 94' der Dichtungseinrichtung 88 ist/sind aus flexiblen Kunststoff, insbesondere aus Silikon, gebildet.

Das Gehäuse 32 des Getriebes 30 und/oder das Zentrier- und/oder Führungselement und/oder das Abtriebszahnrad 42 und/oder das bzw. die zwei Antriebszahnräder 44, 46 und/oder das Trägerelement 56 und/oder die Antriebsbuchsen 66, 66' und/oder die Kupplungselemente 70, 70' und/oder die Dichtungseinrichtung 88 und/oder Gehäuseelement 96 und/oder das Verschlusselement 98 des Gehäuses 92 und/oder die Klemmscheibe 120 ist/sind aus Kunststoff, insbesondere Polyoximethylen, Polyester, Polyphenylensulfon, ABS, Acryl, Polycarbonat, Tetrafluorethylen oder Impax, duroplastischen Elastomeren, mit oder ohne Glasfaserverstärkung, oder einer Kombination hieraus gebildet.

Anstelle dessen kann/können die das Gehäuse 32 des Getriebes 30 und/oder das Zentrier- und/oder Führungselement und/oder das Abtriebszahnrad 42 und/oder das bzw. die zwei Antriebszahnräder 44, 46 und/oder das Trägerelement 56 und/oder die Antriebsbuchsen 66, 66' und/oder die Kupplungselemente 70, 70' und/oder die Dichtungseinrichtung 88 und/oder Gehäuseelement 96 und/oder das Verschlusselement 98 des Gehäuses 92 und/oder die Klemmscheibe 120 alternativ und/oder kumulativ aus Metall, insbesondere (nicht-rostendem) Stahl, Edelstahl, Aluminium, Messing, Zink, Rotgusslegierungen oder einer Legierung hieraus gebildet sein. Demgegenüber sind die Grundkörper 14, 14' der Schneideeinrichtungen 12, 12' und/oder die Kupplungseinrichtung 72 vorzugsweise in aller Regel aus Metall, insbesondere (nicht-rostendem) Stahl, Edelstahl, Aluminium, Messing, Zink, Rotgusslegierungen oder einer Legierung hieraus gebildet.

Ohne im Einzelnen gezeigt zu sein, kann das Getriebe 30, das zwischen den wenigstens zwei Schneideeinrichtungen 12, 12' und der Antriebseinrichtung 22 angeordnet ist, mit dessen einzelnen Bauteilen in Form eines Baukastens ausgebildet und zusammenbaubar sein. Das Getriebe 30 und damit die gesamte Vorrichtung 10 lassen sich auf diese Weise baukastenähnlich vom Operateur oder dem Bedienungspersonal zusammensetzen. Einerseits können somit zusätzliche Montagekosten von Seiten des Herstellers eingespart werden. Andererseits lässt sich die erfindungsgemäße Vorrichtung dadurch einfach und schnell sowie zuverlässig, etwa zu Zwecken einer Desinfektion oder dergleichen, auseinanderbauen und anschließend bei Wiederverwendung erneut montieren. Von Vorteil ist dabei, dass das Gehäuse 32 des Getriebes 30 bzw. das Gehäuseelement 34 und der Gehäusedeckel 36 und/oder das Abtriebszahnrad 42 und/oder das bzw. die zwei Antriebszahnräder 44, 46 und/oder das Trägerelement 56 in Form eines Baukastens ausgebildet und zusammenbaubar ist/sind.

Die Erfindung ist nicht auf die dargestellten Ausführungsformen der Vorrichtung 10 entsprechend den Fig. 1 bis 8 beschränkt. Demnach ist es ohne weiteres möglich, die wenigstens zwei Schneideeinrichtungen 12, 12' zu dem dargestellten Ausführungsbeispiel der erfindungsgemäßen Vorrichtung 10 nicht bzw. nicht ausschließlich im Gegenlauf, sondern alternativ oder kumulativ im Gleichlauf anzutreiben, sofern ein operativer Eingriff einen solchen gleichgerichteten Antrieb erforderlich machen würde. Ohne im Einzelnen dargestellt zu sein, wäre in diesem Fall an sich allgemein bekannt, zusätzliche konstruktive Vorkehrungen im Hinblick auf das Getriebe 30, Schaltungsmöglichkeiten für das Getriebe 30 etc. vorzusehen. Weiterhin kann anstelle einer Gewindeverbindung auch eine Rast- oder Schnappverbindung (nicht im Einzelnen dargestellt) zur gegenseitigen Befestigung von Gehäuseelement 96 und Verschlusselement 98 vorgesehen sein. Dabei sind das Gehäuseelement 96 und das Verschlusselement 98 über wenigstens einen Rastvorsprung oder dergleichen Rastnase an dem Gehäuseelement 96 bzw. an dem Verschlusselement 98 und mindestens einen weiteren Rastvorsprung an dem Verschlusselement 98 bzw. an dem Gehäuseelement 96 miteinander verbunden. Die beiden Rastvorsprünge oder dergleichen Rastnasen wirken zusammen und sind in gegenseitigen Eingriff bringbar. Vorzugsweise kann/können der wenigstens eine Rastvorsprung oder dergleichen Rastnase und/oder der mindestens eine weitere Rastvorsprung oder dergleichen Rastnase in diesem Zusammenhang derart ausgebildet sein, dass das Gehäuseelement 96 und das Verschlusselement 98 des Gehäuses 92 nach Ineingriffbringung miteinander unlösbar verbunden sind oder nach anschließender Außereingriffbringung miteinander unverbindbar sind. Beispielsweise kann/können einer oder beide der Rastvorsprünge oder dergleichen Rastnase/n mit einer Sollbruchstelle versehen sein, um die Vorrichtung 10 nach einer Außereingriffbringung dauerhaft zu beschädigen und damit für eine erneute Verwendung unbrauchbar zu machen. Die Vorrichtung 10 nach der Erfindung eignet sich gleichermaßen gut zum Einmal- oder Mehrfach-Gebrauch.

## Patentansprüche

1. Vorrichtung zum Ausschneiden und Entfernen von Gewebezylindern aus einem Gewebe, das sich innerhalb einer Körper- oder Gelenkhöhle und/oder in bzw. an einem Wandbereich davon befindet, die zwei Schneideeinrichtungen (12, 12') mit jeweils einem hohlzylindrischen Grundkörper (14, 14'), einer distalen Öffnung (18, 18') an einem distalen Ende (16, 16') des Grundkörpers (14, 14') und einem die distale Öffnung (18, 18') umgebenden Schneideelement (20, 20'), die ineinander angeordnet und zueinander relativ drehbar ausgebildet sind, und ein von einem Gehäuse (32) aufgenommenes Getriebe (30), das über eine Kupplungseinrichtung (88) mit mindestens einer Antriebseinrichtung (22) zum Drehen des jeweiligen Grundkörpers (14, 14') der zwei Schneideeinrichtungen (12, 12') um deren Längsachsen (24, 24') in gegenseitigen Wirkeingriff bringbar ist, umfasst, wobei das Getriebe (30) ein Abtriebszahnrad (42), das der Antriebseinrichtung (22) zugeordnet ist, und zwei, mit dem einen Abtriebszahnrad (42) in Wirkeingriff stehende Antriebszahnräder (44, 46), die jeweils einer der zwei Schneideeinrichtungen (12, 12') zugeordnet sind, umfasst, **dadurch gekennzeichnet, dass** das Abtriebszahnrad (42) und die zwei Antriebszahnräder (44, 46) des Getriebes (30) als Kegelräder ausgebildet sind, wobei das Abtriebszahnrad (42) an einem Trägerelement (56), das an dem Gehäuse (32) des Getriebes (30) drehbar abgestützt ist, lösbar befestigt ist und wobei das Trägerelement (56) mit der Antriebseinrichtung (22) über ein zapfenförmiges Übertragungselement (64) mit einem im Querschnitt rechteckförmigen, quadratischen, sechseckförmigen oder sonstwie polygonalen, elliptischen, trapezoiden, parallelogrammartigen oder halbkreisförmigen Profil drehfest verbindbar ist.

2. Vorrichtung nach Anspruch 1, **dadurch gekennzeichnet, dass** die innere Schneideeinrichtung (12) von der benachbarten äußeren Schneideeinrichtung (12') der zwei Schneideeinrichtungen (12, 12') koaxial, insbesondere mit geringem Spiel, aufgenommen ist.

3. Vorrichtung nach Anspruch 1 oder 2, **dadurch gekennzeichnet, dass** die innere Schneideeinrichtung (12) von der benachbarten äußeren Schneideeinrichtung (12') der zwei Schneideeinrichtungen (12, 12') mit größerem Spiel aufgenommen ist, wobei wenigstens ein Zentrier- und/oder Führungselement zwischen den Grundkörpern (14, 14') der inneren Schneideeinrichtung (12) und der benachbarten äußeren Schneideeinrichtung (12') angeordnet ist/sind.

4. Vorrichtung nach einem der Ansprüche 1 bis 3, **dadurch gekennzeichnet, dass** Trägerelement (56) des Abtriebszahnrades (42) an dem Gehäusedeckel (36) des Gehäuses (32) in einer Ausnehmung (61) drehbar abgestützt ist.

5. Vorrichtung nach einem der Ansprüche 1 bis 4, **dadurch gekennzeichnet, dass** das Abtriebszahnrad (44) und das Trägerelement (56) über eine Rast- oder Schnappverbindung oder Gewindeverbindung miteinander lösbar verbunden sind.

6. Vorrichtung nach einem der Ansprüche 1 bis 5, **dadurch gekennzeichnet, dass** die zwei Antriebszahnräder (44, 46) jeweils an Antriebsbuchsen (66, 66'), insbesondere über eine Gewindeverbindung oder Rast- oder Schnappverbindung, befestigt sind.

7. Vorrichtung nach einem der Ansprüche 1 bis 6, **dadurch gekennzeichnet, dass** die Antriebseinrichtung (22) mittels einer, insbesondere elektronischen, Steuer- und/oder Regeleinrichtung steuerbar und/oder regelbar ist, derart, dass die zwei Schneideeinrichtungen (12, 12') durch die Antriebseinrichtung (22) jeweils intervallartig in entgegengesetzter Antriebsrichtung antreibbar sind.

8. Vorrichtung nach Anspruch 7, **dadurch gekennzeichnet, dass** die Zeitintervalle zum Antrieb der zwei Schneideeinrichtungen (12, 12') in entgegengesetzter Antriebsrichtung durch die, insbesondere elektronische, Steuer- und/oder Regeleinrichtung beliebig einstellbar und/oder voreinstellbar sind.

9. Vorrichtung nach einem der Ansprüche 1 bis 8, **dadurch gekennzeichnet, dass** die zwei Schneideeinrichtungen (12, 12') jeweils an dem proximalen Ende (26, 26') und/oder an dem dem proximalen Ende (26, 26') zugewandten Bereich (28, 28') des Grundkörpers (14, 14') mit Kupplungselementen (70, 70') versehen sind, die mit dem jeweiligen Grundkörper (14, 14') unlösbar verbunden sind.

10. Vorrichtung nach einem der Ansprüche 1 bis 9, **dadurch gekennzeichnet, dass** die zwei Schneideeinrichtungen (12, 12') und die Antriebseinrichtung (22) mit dem Getriebe (30) jeweils über eine Kupplungseinrichtung (72) drehfest verbindbar sind.

11. Vorrichtung nach Anspruch 10, **dadurch gekennzeichnet, dass** die Kupplungseinrichtung (72) als Steck-, Rast- oder Schnappeinrichtung ausgebildet ist.

12. Vorrichtung nach Anspruch 10 oder 11, **dadurch gekennzeichnet, dass** die Kupplungseinrichtung (72) einen Kipphebel (74) aufweist, der jeweils an den Antriebsbuchsen (66, 66') und dem Gehäuse (32) des Getriebes (30) um eine Drehachse (76) verschwenkbar angeordnet ist, an einem Ende (78) durch eine Feder (80) beaufschlagt ist und an einem dem einen Ende gegenüberliegenden Ende (82) einen etwa radial angeordneten Stift (84), Bolzen oder Vorsprung zum Eingreifen in einer korrespondierend ausgebildeten Bohrung (86) oder Ausnehmung an dem Kupplungselement (70, 70') der zwei Schneideeinrichtungen (12, 12') bzw. der Antriebseinrichtung (22) umfasst.

13. Vorrichtung nach einem der Ansprüche 9 bis 12, **dadurch gekennzeichnet, dass** das Kupplungselement (70) der inneren/innersten der zwei Schneideeinrichtungen (12, 12') als eine Dichtungseinrichtung (88) zum Verschließen und Freigeben einer proximalen Öffnung an dem proximalen Ende (26) des Grundkörpers (14) ausgebildet ist.

14. Vorrichtung nach Anspruch 13, **dadurch gekennzeichnet, dass** die Dichtungseinrichtung (88) ein zweigeteiltes im Wesentlichen topfförmig ausgebildetes Gehäuse (92) mit einem an dem Grundkörper (14) angebrachten Gehäuseelement (96) und einem an dem Gehäuseelement (96) befestigbaren deckel- oder kappenförmigen Verschlusselement (98) aufweist und zwei Dichtungselemente (94, 94') umfasst, die in dem Gehäuse (92) auf der Längsachse (24) des Grundkörpers (14) der Schneideeinrichtung (12) zueinander benachbart angeordnet sind und miteinander zusammenwirken.

15. Vorrichtung nach Anspruch 14, **dadurch gekennzeichnet, dass** eines (94) der zwei Dichtungselemente (94, 94') der Dichtungseinrichtung (88) im Wesentlichen kegel-, kegelstumpf-, topf-, hut- oder dergleichen -förmig ausgebildet ist, eine Schlitzöffnung (106), die in einer Ebene (108), welche gegenüber einer durch die Grundfläche (110) des Dichtungselementes (94) aufgespannten Ebene (112) in Richtung des distalen Endes (16) des Grundkörpers (14) der Schneideeinrichtung (12) versetzt angeordnet ist, umfasst und einen sich radial nach außen erstreckenden Umfangsabschnitt (114) in der durch die Grundfläche (110) des Dichtungselementes (94) aufgespannten Ebene (112) zum Fixieren in dem Gehäuse (92) aufweist.

16. Vorrichtung nach Anspruch 14 oder 15, **dadurch gekennzeichnet, dass** ein anderes (94') der zwei Dichtungselemente (94, 94') der Dichtungseinrichtung (88) scheibenförmig ausgebildet und eine mittige, etwa kreisförmige Ausnehmung (118), durch welche ein weiteres Instrument, insbesondere ein Greif- oder sonstiges Schneideinstrument, vorzugsweise ein Krallengeifer oder ein Myombohrer, hindurchführbar ist, aufweist.

17. Vorrichtung nach einem der Ansprüche 1 bis 16, **dadurch gekennzeichnet, dass** die Schneideelemente (20, 20') der zwei Schneideeinrichtungen (12, 12') im Wesentlichen angefast, kegelförmig, konisch oder schräg von der distalen Öffnung (18, 18') abgewandt nach außen und/oder zu der distalen Öffnung (18, 18') zugewandt nach innen verlaufend ausgebildet sind.

18. Vorrichtung nach einem der Ansprüche 1 bis 17, **dadurch gekennzeichnet, dass** die Schneideelemente (20, 20') der zwei Schneideeinrichtungen (12, 12') einen umlaufenden oder diskontinuierlichen Glattschliff, Wellenschliff oder Zahnschliff aufweisen.

19. Vorrichtung nach einem der Ansprüche 1 bis 18, **dadurch gekennzeichnet, dass** das zwischen den zwei Schneideeinrichtungen (12, 12') und der Antriebseinrichtung (22) angeordnete Getriebe (30) mit dessen einzelnen Bauteilen in Form eines Baukastens ausgebildet und zusammenbaubar ist.

## Claims

1. Device for excising and removing cylinders of tissue from tissue within a body cavity or articular cavity and/or in the wall area of same, that includes two cutting devices (12, 12') each with a hollow cylindrical main body (14, 14'), a distal opening (18, 18') at a distal end (16, 16') of the main body (14, 14') and a cutting element (20, 20') surrounding the distal opening (18, 18'), that are arranged within each other and designed to rotate relative to each other, and a gear device (30) housed in a housing (32), which by means of a coupling device (88) can be brought into mutual effective engagement with at least one drive device (22) for rotating the respective main body (14, 14') of the two cutting devices (12, 12') about their longitudinal axes (24, 24'), with the gear device (30) comprises an output drive gearwheel (42), which is assigned to the drive device (22), and two input drive gearwheels (44, 46) being in effective engagement with the output drive gearwheel (42), which are each assigned to one of the two cutting devices (12, 12'), **characterized in that** the output drive gearwheel (42) and the two input drive gearwheels (42, 44) of the gear device (30) being designed as bevel gears, with the output drive gearwheel (42) being detachably mounted on a supporting element (56), which is rotatably supported on the housing (32) of the gear device (30), and with the supporting element (56) being twist-proof connectable to the drive device (22) by means of a spigot-shaped transmission element (64) that has a profile which in cross-section is rectangular, square, hexagonal, or of other polygonal, elliptical, trapezoidal, parallelogram-type or semi-circular shape.

2. Device according to Claim 1, **characterized in that** the inner cutting device (12) is coaxially held, in particular with a slight clearance, by the adjacent outer cutting device (12') of the two cutting devices (12, 12').

3. Device according to Claim 1 or 2, **characterized in that** the inner cutting device (12) is held with a larger clearance from the adjacent outer cutting device (12') of the two cutting devices (12, 12'), with at least one centering and/or guiding element being arranged between the basic bodies (14, 14') of the inner cutting device (12) and the adjacent outer cutting device (12').

4. Device according to one of Claims 1 to 3, **characterized in that** the supporting element (56) of the output drive gearwheel (42) is rotatably supported on the housing (32) of the gear device (30) in a recess (61).

5. Device according to one of Claims 1 to 4, **characterized in that** the output drive gearwheel (44) and the supporting element (56) are detachably fixed to each other by means of a latching or snap-on connection or a threaded connection.

6. Device according to one of Claims 1 to 5, **characterized in that** the two input drive gearwheels (44, 46) are each attached to drive bushes (66, 66'), in particular by means of a threaded connection or a latching or snap-on connection.

7. Device according to one of Claims 1 to 6, **characterized in that** the drive device (22) can be controlled (closed-circuit control and/or open-circuit control) by a, in particular electronic, controller in such a way that the two cutting devices (12, 12') can be driven by the drive device (22) at intervals in each case in the opposite drive direction.

8. Device according to Claim 7, **characterized in that** the time intervals for driving the two cutting devices (12, 12') in an opposite drive direction can be set and/or preset as required by a, especially electronic, controller.

9. Device according to one of Claims 1 to 8, **characterized in that** the two cutting devices (12, 12') are each provided with coupling elements (70, 70'), which are undetachably connected to the respective main body (14, 14'), at the proximal end (26, 26') and/or at the area (28, 28') of the main body (14, 14') facing towards the proximal end (26, 26').

10. Device according to one of Claims 1 to 9, **characterized in that** the two cutting devices (12, 12') and the drive device (22) each are twist-proof connectable to the gear device (30) by a coupling device (72).

11. Device according to Claim 10, **characterized in that** the coupling device (72) is designed as a plug-in, detent or snap-on device.

12. Device according to Claim 10 or 11, **characterized in that** the coupling device (72) has a tilting lever (74), that is arranged on the drive bushes (66, 66') and the housing (32) of the gear device (30) so that it can be swivelled about a rotary axis (76), at one end (78) is subjected to the force of a spring (80) and at one end (82) opposite to the one end comprises an approximately radially arranged pin (84), spigot or projection for engaging in a corresponding-shaped bore (86) or recess in the coupling element (70, 70') of the two cutting devices (12, 12') or of the drive device (22).

13. Device according to one of Claims 9 to 12, **characterized in that** the coupling element (70) of the inner/innermost of the two cutting devices (12, 12') is designed as a sealing device (88) for closing and releasing a proximal opening at the proximal end (26) of the main body (14).

14. Device according to Claim 13, **characterized in that** the sealing device (88) has a two-part, essentially pot-shaped, housing (92) with a housing element (96) fitted to the main body (14) and a cover or cap-shaped closing element (98) that can be fitted to the housing element (96), and comprises two sealing elements (94, 94') that are arranged adjacent to each other in the housing (92) on the longitudinal axis (24) of the main body (14) of the cutting device (12), and interact with each other.

15. Device according to Claim 14, **characterized in that** one (94) of the two sealing elements (94, 94') of the sealing device (88) is essentially formed in the shape of a cone, truncated cone, pot, hat or similar, has a slot opening (106) that is in a plane (108) that is arranged relative to a plane (112) extending through the base surface (110) of the sealing element (94) in the direction of the distal end (16) of the main body (14) of the cutting device (12) and has a circumferential section (114), extending radially outwards in the plane (112) extending through the base surface (110) of the sealing element (94), for fixing in the housing (92).

16. Device according to Claim 14 or 15, **characterized in that** another (94') of the two sealing elements (94, 94') of the sealing device (88) is shaped in the form of a disk and has a central, approximately circular, recess (118) through which a further instrument, especially a gripping or other cutting instrument, preferably a claw gripper or a myoma drill, can be inserted.

17. Device according to one of Claims 1 to 16, **characterized in that** the cutting elements (20, 20') of the two cutting devices (12, 12') are essentially chamfered, tapered, conical or sloping, facing outwards away from the distal opening (18, 18') and/or running inwards towards the distal opening (18, 18').

18. Device according to one of Claims 1 to 17, **characterized in that** the cutting elements (20, 20') of the two cutting devices (12, 12') are ground either circumferentially or discontinuously with a smooth edge, wavy edge or toothed edge.

19. Device according to one of Claims 1 to 18, **characterized in that** the gear device (30) arranged between the two cutting devices (12, 12') and the drive device (22) is designed and can be assembled with its individual components in the form of a module.

## Revendications

1. Dispositif pour découper et enlever des cylindres de tissus à partir d'un tissu qui se trouve à l'intérieur d'une cavité corporelle ou articulaire et/ou dans ou sur une zone de paroi de celle-ci, qui comprend deux moyens de coupe (12, 12') avec chacun un corps de base (14, 14') cylindrique creux, une ouverture distale (18, 18') à une extrémité distale (16, 16') du corps de base (14, 14'), et un élément de coupe (20, 20') qui entoure l'ouverture distale (18, 18'), lesquels sont agencés l'un dans l'autre et réalisés rotatifs l'un par rapport à l'autre, et un mécanisme (30) reçu par un boîtier (32), lequel peut être amené en engagement d'action réciproque avec au moins un moyen d'entraînement (22) pour faire tourner les corps de base (14, 14') respectifs des deux moyens de coupe (12, 12') autour de leurs axes longitudinaux, le mécanisme (30) comprenant un engrenage mené (42), qui est associé au moyen d'entraînement (22), et deux engrenages d'entraînement (44, 46) en engagement d'action avec ledit engrenage mené (42), qui sont associés chacun à l'un des deux moyens de coupe (12, 12'), **caractérisé en ce que** l'engrenage mené (42) et les deux engrenages d'entraînement (44, 46) du mécanisme (30) sont réalisés sous forme de pignons coniques, de sorte que l'engrenage mené (42) est fixé de façon détachable sur un élément porteur (56) qui est soutenu en rotation sur le boîtier (32) du mécanisme (30), et l'élément porteur (56) est susceptible d'être relié solidairement en rotation avec le moyen d'entraînement (22) via un élément de transmission (64) en forme de tenon avec une section rectangulaire, carrée, hexagonale ou généralement polygonale, elliptique, trapézoïdale, semblable à un parallélogramme ou en forme de demi-cercle.

2. Dispositif selon la revendication 1, **caractérisé en ce que** le moyen de coupe intérieur (12) est reçu de manière coaxiale, en particulier avec un faible jeu, par le moyen de coupe extérieur voisin (12') des deux moyens de coupe (12, 12').

3. Dispositif selon la revendication 1 ou 2, **caractérisé en ce que** le moyen de coupe intérieur (12) est reçu avec un jeu important par le moyen de coupe extérieur voisin (12') des deux moyens de coupe (12, 12'), et au moins un élément de centrage et/ou de guidage est agencé entre les corps de base (14, 14') du moyen de coupe intérieur (12) et du moyen de coupe extérieur voisin (12').

4. Dispositif selon l'une des revendications 1 à 3, **caractérisé en ce que** l'élément porteur (56) de l'engrenage mené (42) est monté rotatif dans un évidement (61) sur le couvercle (36) du boîtier (32).

5. Dispositif selon l'une des revendications 1 à 4, **caractérisé en ce que** l'engrenage mené (44) et l'élément porteur (56) sont reliés l'un à l'autre de façon détachable via une liaison à enclenchement ou à encliquetage ou encore une liaison à vis.

6. Dispositif selon l'une des revendications 1 à 5, **caractérisé en ce que** les deux engrenages d'entraînement (44, 46) sont fixés respectivement sur des douilles d'entraînement (66, 66'), en particulier via une liaison à vis ou une liaison à enclenchement ou à encliquetage.

7. Dispositif selon l'une des revendications 1 à 6, **caractérisé en ce que** le moyen d'entraînement (22) peut être commandé et/ou régulé au moyen d'une unité de commande et/ou de régulation, en particulier électronique, de telle façon que les deux moyens de coupe (12, 12') peuvent être entraînés par le moyen d'entraînement (22) respectivement par intervalles dans des directions d'entraînement opposées.

8. Dispositif selon la revendication 7, **caractérisé en ce que** les intervalles temporels pour l'entraînement des deux moyens de coupe (12, 12') en directions d'entraînement opposées sont susceptibles d'être réglés/ou préréglés de façon arbitraire au moyen de l'unité de commande et/ou de régulation, en particulier électronique.

9. Dispositif selon l'une des revendications 1 à 8, **caractérisé en ce que** les deux moyens de coupe (12, 12') sont dotés chacun à l'extrémité proximale (26, 26') et/ou au niveau de la zone (28, 28') du corps de base (14, 14') tournée vers l'extrémité proximale (26, 26'), d'éléments d'accouplement (70, 70') qui sont reliés de façon indémontable avec le corps de base respectif (14, 14').

10. Dispositif selon l'une des revendications 1 à 9, **caractérisé en ce que** les deux moyens de coupe (12, 12') et le moyen d'entraînement (22) sont susceptibles d'être reliés solidairement en rotation au mécanisme (30) via un moyen d'accouplement respectif (72).

11. Dispositif selon la revendication 10, **caractérisé en ce que** le moyen d'accouplement (72) est réalisé sous forme de moyen à enfichage, à enclenchement ou à encliquetage.

12. Dispositif selon la revendication 10 ou 11, **caractérisé en ce que** le moyen d'accouplement (71) comprend un levier basculant (74) qui est agencé respectivement sur les douilles d'entraînement (66, 66') et sur le boîtier (32) du mécanisme (30) avec possibilité de pivoter autour d'un axe de rotation (76), qui est sollicité à une extrémité (78) par un ressort (80) et qui comprend à une autre extrémité (82) à l'opposé de la première extrémité, une tige (84), un goujon ou une saillie, agencé(e) approximativement radialement, destiné(e) à s'engager dans un perçage (86) ou un évidement réalisé en correspondance sur l'élément d'accouplement (70, 70') des deux moyens de coupe (12, 12') ou du moyen d'entraînement (22).

13. Dispositif selon l'une des revendications 9 à 12, **caractérisé en ce que** l'élément d'accouplement (70) du moyen de coupe intérieur des deux moyens de coupe (12, 12') est réalisé sous la forme d'un moyen à étanchement (88) pour obturer et dégager une ouverture proximale à l'extrémité proximale (26) du corps de base (14).

14. Dispositif selon la revendication 13, **caractérisé en ce que** le moyen à étanchement (88) comprend un boîtier (92) en deux parties réalisé sensiblement en forme de pot, avec un élément de boîtier (96) monté sur le corps de base (14) et avec un élément d'obturation (98) en forme de couvercle ou de capot, susceptible d'être fixé sur l'élément de boîtier (96), et deux éléments d'étanchéité (94, 94') qui sont agencés au voisinage l'un de l'autre dans le boîtier (92) sur l'axe longitudinal (24) du corps de base (14) du moyen de coupe (12) et qui coopèrent l'un avec l'autre.

15. Dispositif selon la revendication 14, **caractérisé en ce que** l'un (94) des deux éléments d'étanchéité (94, 94') du moyen à étanchement (88) est réalisé sensiblement en forme de cône, de tronc de cône, de pot, de chapeau ou similaire, comprend une ouverture en fente (106) agencée dans un plan (108) qui est décalé par rapport au plan (112) défini par la surface de base (110) de l'élément d'étanchéité (94) en direction de l'extrémité distale (16) du corps de base (14) du moyen de coupe (12), et qui comprend un tronçon périphérique (114) s'étendant radialement vers l'extérieur dans le plan (112) défini par la surface de base (110) de l'élément d'étanchéité (94) pour la fixation dans le boîtier (92).

16. Dispositif selon la revendication 14 ou 15, **caractérisé en ce qu'**un autre (94') des deux éléments d'étanchéité (94, 94') du moyen à étanchement (88) est réalisé en forme de disque et comporte une ouverture centrale (118), approximativement en forme de cercle, à travers laquelle il est possible de faire passer un autre instrument, en particulier un instrument de préhension ou un autre instrument de coupe, de préférence un instrument de préhension à griffe ou une mèche pour myomes.

17. Dispositif selon l'une des revendications 1 à 16, **caractérisé en ce que** les éléments de coupe (20, 20') des deux moyens de coupe (12, 12') sont réalisés sensiblement avec un chanfrein, en forme de coin, en forme de cône ou en oblique en se détournant vers l'extérieur depuis l'ouverture distale (18, 18') et/ou de manière à s'étendre vers l'intérieur en étant tournés vers l'ouverture distale (18, 18').

18. Dispositif selon l'une des revendications 1 à 17, **caractérisé en ce que** les éléments de coupe (20, 20') des deux moyens de coupe (12, 12') comprennent une finition meulée lisse, ondulée ou dentée, périphérique ou discontinue.

19. Dispositif selon l'une des revendications 1 à 18, **caractérisé en ce que** le mécanisme (30) agencé entre les deux moyens de coupe (12, 12') et le moyen d'entraînement (22) est réalisé avec ses composants individuels sous la forme d'un jeu de construction et susceptible d'être assemblé à partir de ces composants.
